(19) Europäisches Patentamt
European Patent Office
Office européen des brevets

(11) EP 4 542 980 A1

(12) **EUROPEAN PATENT APPLICATION**
published in accordance with Art. 153(4) EPC

(43) Date of publication:
**23.04.2025 Bulletin 2025/17**

(21) Application number: **23823557.6**

(22) Date of filing: **02.05.2023**

(51) International Patent Classification (IPC):
***H04N 5/66*** *(2006.01)* ***A61B 5/11*** *(2006.01)*
***A61B 10/00*** *(2006.01)* ***B60R 11/02*** *(2006.01)*
***G03B 21/14*** *(2006.01)* ***G09G 5/00*** *(2006.01)*
***G09G 5/377*** *(2006.01)* ***H04N 5/74*** *(2006.01)*

(52) Cooperative Patent Classification (CPC):
**A61B 5/11; A61B 10/00; B60R 11/02; G03B 21/14; G09G 5/00; G09G 5/377; H04N 5/66; H04N 5/74**

(86) International application number:
**PCT/JP2023/017212**

(87) International publication number:
**WO 2023/243254 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.06.2022 JP 2022096132**

(71) Applicant: **Sony Group Corporation
Tokyo 108-0075 (JP)**

(72) Inventors:
- **KUBOTA, Hiroyuki
Tokyo 108-0075 (JP)**
- **TAKEUCHI, Hiroshi
Tokyo 108-0075 (JP)**
- **TAKAMATSU, Takashi
Tokyo 108-0075 (JP)**
- **TOMONAGA, Seishi
Tokyo 108-0075 (JP)**
- **IMAMURA, Hiroshi
Tokyo 108-0075 (JP)**
- **YOSHITOMI, Kosuke
Tokyo 108-0075 (JP)**

(74) Representative: **Witte, Weller & Partner
Patentanwälte mbB
Postfach 10 54 62
70047 Stuttgart (DE)**

(54) **INFORMATION PROCESSING DEVICE, INFORMATION PROCESSING METHOD, AND SYSTEM**

(57) To provide an information processing apparatus that performs processing for preventing or reducing the occurrence of motion sickness of an occupant in a movable object with a display system mounted thereon.

The information processing apparatus includes: a determining unit that determines motion sickness of an occupant in a movable object; and a processing unit that performs, on the basis of a determination result of the determining unit, processing for determining transmittance of at least a partial region with a transmittance variable region in a display system that is mounted on the movable object and displays a video in the transmittance variable region. The processing unit determines the transmittance of the video to be displayed in the transmittance variable region and the processing unit further determines a displacement amount of at least one of a display position or an angle of a video in the transmittance variable region.

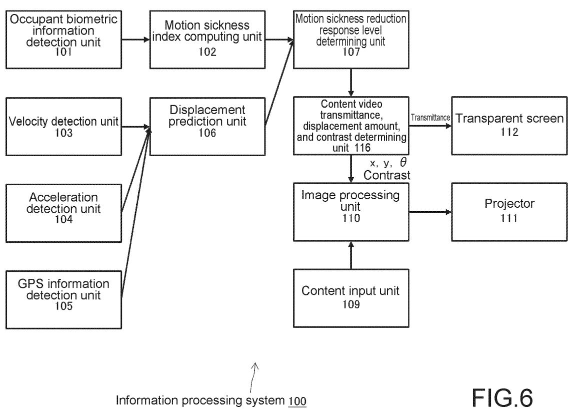


FIG.6

EP 4 542 980 A1

**Description**

Technical Field

A technology disclosed in this specification (hereinafter, referred to as "the present disclosure") relates to an information processing apparatus, an information processing method, and a system that perform processing regarding control of a display system mounted on a movable object.

Background Art

There are needs to view a video on a large display in order to obtain a sense of immersion even in a vehicle. For example, at Level 4 (complete automated driving under a particular condition such as a city) and Level 5 (complete automated driving) of automated driving, needs for an occupant to view a video increase. However, if the display occupies the field-of-view, the field-of-view information in front of the vehicle is lost, and it is thus difficult for the occupant to recognize car behaviors. There is a problem that it becomes difficult to match the body to the motion of the vehicle, and motion sickness including motion sickness caused by a vehicle and the like easily occurs as a result.

For example, a video display apparatus capable of reducing a burden on a passenger and reducing the occurrence of motion sickness by matching visual sense information with vestibule information and bodily sense information based on motion of a vehicle by imparting a visual guidance self-movement feeling while the passenger watches television or the like, on the vehicle, has been proposed (see Patent Literature 1). The video display apparatus displays a video of a camera in the front of the vehicle in a peripheral region out of a display region of the display and displays content in a central region, thereby reducing the motion sickness, but the content display region is not used for recognizing the front.

Moreover, an image processing apparatus that predicts a displacement of a movable object after a predetermined time, crops an image obtained by imaging the outside of a movable object on the basis of the predicted displacement, outputs it as a display image, thereby giving a visual guidance self-movement feeling so that it can be seen in continuation with the actual landscape for reducing motion sickness has been proposed (see Patent Literature 2). This image processing apparatus determines a content display position and an angle of rotation from the prediction of the displacement of the movable object, but the content display region is not used for recognizing the front.

Moreover, a display apparatus that prevents or reduces the occurrence of motion sickness and reduces symptoms of the motion sickness by displaying a predetermined graphic image by deforming it in an orientation of deformation corresponding to a direction opposite to a direction of acceleration according to an inertial force and tilting an image display unit or the predetermined graphic image to the direction opposite to the direction of acceleration according to the inertial force has been proposed (see Patent Literature 3). This display apparatus rotates the content and guides the user's posture, but the display image is not used for recognizing the front.

Citation List

Patent Literature

Patent Literature 1: Japanese Patent Application Laid-open No. 2008-242251
Patent Literature 2: Japanese Patent Application Laid-open No. 2012-19452
Patent Literature 3: Japanese Patent Application Laid-open No. 2019-174523

Disclosure of Invention

Technical Problem

It is an objective of the present disclosure to provide an information processing apparatus, an information processing method, and a system that perform processing for preventing or reducing the occurrence of motion sickness of an occupant in a movable object with a display system mounted thereon. Solution to Problem

The present disclosure has been made in view of the above-mentioned problem and a first aspect thereof is an information processing apparatus including:

a determining unit that determines motion sickness of an occupant in a movable object; and
a processing unit that performs, on the basis of a determination result of the determining unit, processing for determining transmittance of at least a partial region with a transmittance variable region in a display system that is mounted on the movable object and displays a video in the transmittance variable region.

The determining unit determines a response level of the motion sickness on the basis of a combination of a motion sickness index of the occupant and a predicted displacement of the movable object. Then, the processing unit determines transmittance of at least a partial region of the transmittance variable region on the basis of the response level of the motion sickness which is determined by the determining unit. Although the determining unit determines the motion sickness of the occupant on the basis of biometric information of the occupant, the determining unit may be adapted to determine the motion sickness of the occupant on the basis of an accumulation factor and a recovery factor of the motion sickness.
Although the processing unit determines the transmittance of the video to be displayed in the transmittance variable region, the processing unit may further determine a displacement amount of at least one of a display position or an angle of a video in the transmittance variable region.
The display system includes a transparent screen with variable transmittance and a projector that projects a video on the transparent screen. Then, the processing unit determines transmittance of the entire transparent screen or transmittance of the video to be projected on the transparent screen.
Moreover, a second aspect of the present disclosure is an information processing method including:

a determining step of determining motion sickness of an occupant in a movable object; and
a processing step of performing, on the basis of a determination result of the determining step, processing for determining transmittance of at least a partial region with a transmittance variable region in a display system that is mounted on the movable object and displays a video in the transmittance variable region.

Moreover, a third aspect of the present disclosure is a system including:

a display system that is mounted on a movable object and displays a video in a transmittance variable region;
a determining unit that determines motion sickness of an occupant in the movable object; and
a processing unit that performs, on the basis of a determination result of the determining unit, processing for determining transmittance of at least a partial region with a transmittance variable region in the display system.

It should be noted that the "system" set forth therein refers to a logical set of a plurality of apparatuses (or functional modules that realize particular functions), and whether or not the respective apparatuses or the functional modules are in a single casing is not particularly relevant. That is, both a single apparatus consisting of a plurality of components or functional modules and a set of a plurality of apparatuses are equivalent to the "system".

Advantageous Effects of Invention

In accordance with the present disclosure, it is possible to provide an information processing apparatus, an information processing method, and a system that perform processing for preventing or reducing the occurrence of motion sickness of an occupant by adjusting transmittance in a display system that displays a content video in a transmittance variable region mounted on a movable object.
It should be noted that the effects described herein are examples only, and effects provided by the present disclosure are not limited thereto. Moreover, the present disclosure may produce additional effects in addition to the above-mentioned effects.
Other objectives, features, and advantages of the present disclosure will become apparent from more detailed descriptions based on embodiments to be described later and the accompanying drawings.

Brief Description of Drawings

[Fig. 1] Fig. 1 is a diagram showing a functional configuration example of an information processing system 100.
[Fig. 2] Fig. 2 is a diagram showing an example of a determination table.
[Fig. 3] Fig. 3 is a flowchart showing a processing procedure for controlling the display of a content video in accordance with a motion sickness index of an occupant and a predicted displacement of a vehicle.
[Fig. 4] Fig. 4 is a diagram showing a second configuration example of the information processing system 100.
[Fig. 5] Fig. 5 is a diagram showing a third configuration example of the information processing system 100.
[Fig. 6] Fig. 6 is a diagram showing a fourth configuration example of the information processing system 100.
[Fig. 7] Fig. 7 is a diagram showing a fifth configuration example of the information processing system 100.

[Fig. 8] Fig. 8 is a view showing a state in which a content video is displayed by a display system mounted on the vehicle.

[Fig. 9] Fig. 9 is a view showing a state in which the content video is displayed by the display system mounted on the vehicle (an example in which the transmittance is adjusted).

[Fig. 10] Fig. 10 is a view showing a state in which the content video is displayed by the display system mounted on the vehicle (an example in which the transmittance is adjusted).

[Fig. 11] Fig. 11 is a view showing a variation of a arrangement position of a transmittance-variable region in a vehicle-mounted display system.

[Fig. 12] Fig. 12 is a view showing a projector arrangement position corresponding to a transparent screen for RSE.

[Fig. 13] Fig. 13 is a view showing a projector arrangement position corresponding to a transparent screen arranged on a front window.

[Fig. 14] Fig. 14 is a view showing a projector arrangement position corresponding to a transparent screen arranged on a rear window.

[Fig. 15] Fig. 15 is a view showing a projector arrangement position corresponding to a transparent screen arranged on a side window.

[Fig. 16] Fig. 16 is a view showing an operation example of the display system for reducing the motion sickness (making the video transparent).

[Fig. 17] Fig. 17 is a view showing an operation example of the display system for reducing the motion sickness (making the video transparent and rotating the video).

[Fig. 18] Fig. 18 is a view showing an operation example of the display system for reducing the motion sickness (making the video transparent and moving the video) .

[Fig. 19] Fig. 19 is a view showing an operation example of the display system for reducing the motion sickness (making the video transparent and reducing the size of the video).

[Fig. 20] Fig. 20 is a view showing an operation example of the display system for reducing the motion sickness (making the video transparent, rotating the video, and moving the video).

[Fig. 21] Fig. 21 is a view showing an operation example of the display system for reducing the motion sickness (making the video transparent, rotating the video, and reducing the size of the video).

[Fig. 22] Fig. 22 is a view showing an operation example of the display system for reducing the motion sickness (making the video transparent, moving the video, and reducing the size of the video).

[Fig. 23] Fig. 23 is a view showing an operation example of the display system for reducing the motion sickness (making the video transparent, rotating the video, moving the video, and reducing the size of the video).

Mode(s) for Carrying Out the Invention

Hereinafter, the present disclosure will be described in the following order with reference to the drawings.

A. Overview of Display System
B. Reduction of Motion Sickness
C. System Configuration Example (1)
D. System Configuration Example (2)
E. System Configuration Example (3)
F. System Configuration Example (4)
G. System Configuration Example (5)

A. Overview of Display System

There are needs to view a video on a large display in order to obtain a sense of immersion in the vehicle. For example, at Level 4 and Level 5 of automated driving, needs for the occupant to view a video increase. In view of this, in the present disclosure, it is envisaged that a display system that displays a content video in a transmittance variable region is mounted on the vehicle.

Specifically, the display system that displays a content video in a transmittance variable region is constituted by a combination of a transparent screen and a projector that projects a video on the transparent screen. Here, the transparent screen is configured by, for example, laminating a film with electrically variable transmittance on a transparent film such as a resin. Moreover, the display system that displays the content video in the transmittance variable region may be configured with a display device or the like with a transparent flat panel display (FPD) such as a transparent organic light-emitting diode (OLED) or a transparent liquid crystal display (LCD). Alternatively, the display system may be of a video see-through type that displays a landscape in the front of the vehicle (or the periphery of the vehicle), combined with a vehicle-mounted camera by using a display device of a non-see-through-type, not a see-through-type.

The transmittance variable region of the display system may be a transparent screen or a transparent FPD flipped down from the bottom surface of the cabin roof. Fig. 8 shows a state in which a transparent screen 801 is flipped down from the bottom surface of the cabin roof and a content video 803 is projected to substantially the center of the transparent screen 801 from a projector 802 mounted also on the bottom surface of the roof. The transparent screen 801 of the flipped down type is mounted on the vicinity of the back of the seat of the first row, for example. Therefore, the occupant sitting in the rear seat can view the content video 803 projected to the transparent screen 801. In short, the display system shown in Fig. 8 can be said to be a rear-seats entertainment (RSE) system. It should be noted that it is assumed that the rear seat includes a seat of a second row of a 2-row seat car or a seat of second and third rows of a 3-row seat car.

A film with electrically variable transmittance is laminated on the transparent screen 801, and by electrically controlling the transmittance of this film, it is possible to adjust the transmittance of the entire transparent screen 801 or a part thereof (e.g., only a partial region in which the content video 803 has been projected). Moreover, by adjusting the contrast of the content video 803 projected by the projector 802, it is possible to adjust the transmittance of the display region of the content video 803 (increasing the irradiation intensity to increase the contrast decreases the transmittance and in contrast, decreasing the irradiation intensity to decrease the contrast increases the transmittance).

It can be seen that when the transmittance of the entire transparent screen 801 or a part thereof is increased, the occupant in the rear seat can easily recognize the situation in the front of the vehicle and behaviors of the vehicle through its transparent region. On the other hand, as shown in Fig. 9, the sense of immersion of the content video 803 increases when the transmittance of the entire transparent screen 801 is decreased. As shown in Fig. 10, the sense of immersion further increases when the transmittance of the entire transparent screen 801 is decreased and the display size of the content video 803 is increased.

The same applies to a display system using a transparent FPD. By increasing the transmittance of the entire transparent FPD including the content video or increasing the transmittance of a region other than the display region of the content video, the occupant in the rear seat can easily recognize the situation in the front of the vehicle and behaviors of the vehicle through the transparent region. Moreover, the sense of immersion further increases by decreasing the transmittance of the entire transparent FPD or increasing the display size of the content video.

It should be noted that the above-mentioned display system for RSE is an example and the arrangement position of the transparent screen or the transparent FPD is not limited to that shown in Fig. 8. Fig. 11 shows a variation of the arrangement position of the transmittance variable region such as the transparent screen or the transparent FPD in the vehicle-mounted display system, taking a 3-row seat car as an example.

In Fig. 11, the reference numeral 1101 indicates the arrangement position of the transparent screen for RSE or the transparent FPD at the back of the first row seat (or in the front of the second row seat). The reference numeral 1111 indicates an example in which a front window (wind shield) is set as the arrangement position of the transparent screen or the transparent FPD. The reference numeral 1121 indicates an example in which the rear window is set as the arrangement position of the transparent screen or the transparent FPD. For example, in a case where the seats of the second or third row have been turned backwards, it is sufficient to display a content video for the occupant sitting in the seat facing backwards on the rear window. The reference numerals 1131R, 1132R, and 1133R indicate an example in which the right side windows in the first, second, and third rows are set as arrangement positions of the transparent screen or the transparent FPD, respectively. Moreover, the reference numerals 1131L, 1132L, and 1133L indicate an example in which the left side windows in the first, second, and third rows are set as arrangement positions of the transparent screen or the transparent FPD, respectively.

It should be noted that in a case where the transparent screen or the transparent FPD is arranged on the car window such as the front window, the rear window, or the side window, it is possible to use it as a display and also use it as a smoked glass with adjustable transmittance.

In a case where a transparent screen is used as the transmittance variable region, it is necessary to mount a projector that projects a content video on the transparent screen in the vehicle together with it. The projector arrangement position for each arrangement position of the transparent screen in the vehicle in a case of the display system using the combination of the transparent screen and the projector will be described.

Fig. 12 illustrates an arrangement position of a projector 1201 in a case where a transparent screen 1200 for RSE is arranged at the back of the first row seat. Fig. 13 illustrates an arrangement position of a projector 1301 in a case where a transparent screen 1300 is arranged on the front window. Fig. 14 illustrates an arrangement position of a projector 1401 in a case where a transparent screen 1400 is arranged on the rear window. For example, in a case where the seats of the second or third row have been turned backwards, it is sufficient to display a content video for the occupant sitting in the seat facing backwards on the rear window. Fig. 15 illustrates an arrangement position of a projector 1501 in a case where a transparent screen 1500 is arranged on the side window.

In any example shown in Figs. 12, 14, and 15, the projectors 1201, 1401, and 1501 are each arranged on the bottom surface of the roof. Moreover, in the example shown in Fig. 13, the projector 1301 is arranged in the vicinity of the center directly below a dashboard or a front wind shield. In any arrangement example, by arranging the projector in a position as close as possible to the transparent screen on which a content video is to be projected, it is possible to cause a video to be projected without being blocked by an object (e.g., an occupant) and to keep the maximum luminance high.

B. Reduction of Motion Sickness

For example, in a case where the display system for RSE has been mounted as shown in Fig. 8, the front field-of-view of the occupant sitting in the rear seat is occupied by a display (transparent screen or transparent FPD), and it is difficult to grasp the situation in the front of the vehicle. Therefore, there is a problem that it becomes difficult for the occupant to match the body to the motion of the vehicle, and the motion sickness easily occurs as a result.

In view of this, in the present disclosure, using the display system characteristics of displaying the content video in the transmittance variable region, in other words, of being capable of adjusting the transmittance of the video to be displayed, the motion sickness is reduced by controlling the transmittance of the transmittance variable region in accordance with a response level for motion sickness reduction considering the motion sickness index and the predicted displacement. Specifically, when the response level for motion sickness reduction has increased, the amount of field-of-view information in front of the vehicle through the entire display is increased by increasing the transmittance of the transparent screen or the transparent FPD. Accordingly, the visibility of the content video lowers, but it becomes easy to recognize the situation in the front of the vehicle, and it becomes possible to prevent development of the motion sickness by, for example, the occupant matching the body to the motion of the vehicle or to reduce the symptoms. Moreover, in accordance with the present disclosure, the motion sickness can also be reduced by changing the display position and the angle of the content video in accordance with the response level for motion sickness reduction to makes it easy for the occupant to match the posture to the displacement of the vehicle body.

A display operation realized by the present disclosure will be described, exemplifying a display system for RSE, in other words, a display system mounting a display (e.g., transparent screen or transparent FPD) in the front of the vehicle as shown in Fig. 8. It is envisaged that for example in a case where there is a steep curve on the left-hand side in front of the vehicle, the occupant wishes to recognize the front so that the occupant can easily match the body to the displacement of the vehicle body for reducing the motion sickness. Figs. 16 to 23 each show an operation example of the display system for reducing the motion sickness in such a case.

In the example shown in Fig. 16, the content video 803 displayed at the center of the transparent screen is made transparent. The occupant sitting in the rear seat has lower visibility of the content video 803 as compared to the display example shown in Figs. 8 and 9, and the occupant can easily recognize the front of the vehicle and match the posture of the body to the displacement of the vehicle body, and therefore the motion sickness can be reduced.

In the example shown in Fig. 17, the content video 803 displayed at the center of the transparent screen is made transparent and is rotated in a counter-clockwise direction in accordance with a sharp left turn of the vehicle body. Also in the display example shown in Fig. 17, the occupant sitting in the rear seat can easily recognize the front of the vehicle and match the posture of the body to the displacement of the vehicle body, and therefore the motion sickness can be reduced. Moreover, the content video 803 is rotated in accordance with a tilt angle of the posture of the body which has been made to follow the displacement of the vehicle body during the sharp left turn, and therefore the visibility of the content video 803 is further improved as compared to the display example shown in Fig. 16.

In the example shown in Fig. 18, the content video 803 displayed at the center of the transparent screen is made transparent and is moved in accordance with a sharp left turn of the vehicle body. Also in the display example shown in Fig. 18, the occupant sitting in the rear seat can easily recognize the front of the vehicle and match the posture of the body to the displacement of the vehicle body, and therefore the motion sickness can be reduced. Since the display position of the content video 803 is moved leftwards from the center of the transparent screen, the vehicle front field-of-view is further improved, and the occupant can more easily recognize the displacement of the vehicle body. Moreover, the content video 803 is moved leftwards to be adapted to the position of the body tilted leftwards for withstanding the centrifugal force applied during the sharp left turn, and therefore the visibility of the content video 803 is further improved as compared to the display example shown in Fig. 16.

In the example shown in Fig. 19, the content video 803 displayed at the center of the transparent screen is made transparent and reduced in size. Also in the display example shown in Fig. 19, the occupant sitting in the rear seat can easily recognize the front of the vehicle and match the posture of the body to the displacement of the vehicle body, and therefore the motion sickness can be reduced. Due to the size reduction of the content video 803, the visibility of the content video 803 further lowers, but the vehicle front field-of-view is further improved, and the occupant can more easily recognize the displacement of the vehicle body.

In the example shown in Fig. 8, the content video 803 displayed at the center of the transparent screen is made transparent and is rotated in the counter-clockwise direction and moved leftwards in accordance with a sharp left turn of the vehicle body. Also in the display example shown in Fig. 8, the occupant sitting in the rear seat can easily recognize the front of the vehicle and match the posture of the body to the displacement of the vehicle body, and therefore the motion sickness can be reduced. Since the display position of the content video 803 is moved leftwards from the center of the transparent screen, the vehicle front field-of-view is further improved, and the occupant can more easily recognize the displacement of the vehicle body. Moreover, the content video 803 is rotated in accordance with a tilt angle of the posture of the body which has been made to follow the displacement of the vehicle body during the sharp left turn, and therefore the visibility of the content video 803 is further improved as compared to the display example shown in Fig. 16.

In the example shown in Fig. 21, the content video 803 displayed at the center of the transparent screen is made transparent and is rotated in the counter-clockwise direction and reduced in size in accordance with a sharp left turn of the vehicle body. Also in the display example shown in Fig. 21, the occupant sitting in the rear seat can easily recognize the front of the vehicle and match the posture of the body to the displacement of the vehicle body, and therefore the motion sickness can be reduced. Moreover, the content video 803 is rotated in accordance with a tilt angle of the posture of the body which has been made to follow the displacement of the vehicle body during the sharp left turn, and therefore the visibility of the content video 803 is further improved as compared to the display example shown in Fig. 16. Due to the size reduction of the content video 803, the visibility of the content video 803 further lowers, but the vehicle front field-of-view is further improved, and the occupant can more easily recognize the displacement of the vehicle body.

In the example shown in Fig. 22, the content video 803 displayed at the center of the transparent screen is made transparent and is moved leftwards and reduced in size in accordance with a sharp left turn of the vehicle body. Also in the display example shown in Fig. 22, the occupant sitting in the rear seat can easily recognize the front of the vehicle and match the posture of the body to the displacement of the vehicle body, and therefore the motion sickness can be reduced. Since the display position of the content video 803 is moved leftwards from the center of the transparent screen, the vehicle front field-of-view is further improved, and the occupant can more easily recognize the displacement of the vehicle body. Due to the size reduction of the content video 803, the visibility of the content video 803 further lowers, but the vehicle front field-of-view is further improved, and the occupant can more easily recognize the displacement of the vehicle body. Moreover, the visibility of the content video 803 is improved by an amount corresponding to the left movement of the content video 803 to be adapted to the position of the body tilted leftwards for withstanding the centrifugal force applied during the sharp left turn.

In the example shown in Fig. 23, the content video 803 displayed at the center of the transparent screen is made transparent and is rotated in the counter-clockwise direction and moved leftwards and reduced in size in accordance with a sharp left turn of the vehicle body. Also in the display example shown in Fig. 23, the occupant sitting in the rear seat can easily recognize the front of the vehicle and match the posture of the body to the displacement of the vehicle body, and therefore the motion sickness can be reduced. Moreover, the visibility of the content video 803 is improved by an amount corresponding to the rotation and movement of the content video 803 according to the position and the posture of the body tilted and moved leftwards to follow the displacement of the vehicle body during the sharp left turn. Due to the size reduction of the content video 803, the visibility of the content video 803 further lowers, but the vehicle front field-of-view is further improved, and the occupant can more easily recognize the displacement of the vehicle body.

Fig. 17, Fig. 18, and Figs. 20 to 23 show the examples in which the content video made transparent on the display (e.g., transparent screen or transparent FPD) is rotated leftwards and moved leftwards when the vehicle makes a sharp left turn. Although the illustration is omitted, the effect of reducing the motion sickness of the occupant can also be obtained in such a manner that the content video made transparent on the display is rotated rightwards and moved rightwards when the vehicle makes a sharp right curve.

Moreover, the effect of reducing the motion sickness of the occupant can also be obtained in such a manner that the content video made transparent is moved upwards and downwards, not leftwards and rightwards, when the vehicle passes by a steep slope, not makes a steep curve. For example, when the vehicle goes up a steep ascent, the content video only needs to be moved upwards in accordance with upward movement of the line of sight of the occupant. In contrast, the vehicle goes down a steep descent, the content video only needs to be moved downwards in accordance with downward movement of the line of sight of the occupant.

## C. System Configuration Example (1)

Fig. 1 shows a functional configuration example of an information processing system 100 that performs processing of controlling the transmittance of the transparent screen and the display position and angle of the content video. The information processing system 100 shown in the figure includes an occupant biometric information detection unit 101, a motion sickness index computing unit 102, a velocity detection unit 103, an acceleration detection unit 104, a global positioning system (GPS) information detection unit 105, a displacement prediction unit 106, a motion sickness reduction response level determining unit 107, a content video transmittance and displacement amount determining unit 108, a content input unit 109, an image processing unit 110, a projector 111, and a transparent screen 112. It should be noted that although the information processing system 100 may be configured as a physically single apparatus with a single casing in which those functional blocks 101 to 112 are all stored, each functional block may be configured as respective individual apparatuses or the functional blocks 101 to 112 may be classified into two or more groups and each group may be configured as an apparatus. As an example, the functional blocks 106 to 110 may be configured as a physically single apparatus (in Fig. 1, the functional blocks that can be configured as single apparatuses are surrounded by the dotted line). Moreover, as another example, the functional blocks 106 to 112 may be configured as a physically single apparatus or the projector 111 and the transparent screen 112 of the functional blocks 101 to 112 may be configured as a peripheral apparatus externally connected to the information processing system 100. Moreover, in a case where the information processing system 100 is a vehicle control system (or a part of the vehicle control system) mounted on the vehicle, the occupant biometric information detection unit 101, the velocity detection unit 103, the acceleration detection unit 104, and the GPS information detection unit 105 may be in-vehicle sensors externally connected to the information processing system 100. As a matter of course, the information processing system 100 may further include functional blocks other than the functional blocks 101 to 112 shown in the figure, and, depending on operation or the like of the system, at least some of the functional blocks 101 to 112 may be removed or may be replaced by the functional blocks (not shown).

The occupant biometric information detection unit 101 detects biometric information such as heart beat rate and electroencephalography of the occupant. The occupant biometric information detection unit 101 may set a person who views a content video displayed by the display system (see above) as a detection target and exclude an occupant who does not view the content video from the targets. The motion sickness index computing unit 102 computes a motion sickness index of the occupant on the basis of the biometric information detected by the occupant biometric information detection unit 101.

The velocity detection unit 103 detects velocity information of the vehicle and the acceleration detection unit 104 detects vehicle acceleration information. The velocity detection unit 103 and the acceleration detection unit 104 are, for example, a velocity sensor, an acceleration sensor, an angular velocity sensor (gyro sensor) mounted on the vehicle, or an inertial measurement unit (IMU) integrating them. The GPS information detection unit 105 analyzes a GPS signal received from a GPS satellite, measures the current location of the vehicle, and further detects map information of a driving planned route of the vehicle. The displacement prediction unit 106 computes a predicted displacement of the vehicle, i.e., a displacement amount of the vehicle after a certain time on the basis of the information detected by the respective detection units 103 to 105.

The motion sickness reduction response level determining unit 107 determines a response level for motion sickness reduction from the determination table determined in advance on the basis of the respective information, the motion sickness index computed by the motion sickness index computing unit 102 and the displacement amount predicted by the displacement prediction unit 106.

The determination table set forth herein is a table showing a relationship between a combination of a motion sickness index and a predicted displacement amount and a response level for motion sickness reduction. Fig. 2 shows an example of the determination table. In the present embodiment, the response level for motion sickness reduction is associated with the transmittance of the content video. For example, in a case where the motion sickness index is large and the predicted displacement amount is large, the response level for motion sickness reduction is 90% and the transmittance of the content video is set to be 90%.

The content video transmittance and displacement amount determining unit 108 determines transmittance $\tau$ and a displacement amount $(x, y, \theta)$ of the content video that the projector 111 projects onto the transparent screen 112 on the basis of the response level determined by the motion sickness reduction response level determining unit 107. Moreover, the content video transmittance and displacement amount determining unit 108 may be further adapted to also determine a display size of the content video.

A video signal of the content that the projector 111 projects onto the transparent screen 112 is input to the image processing unit 110 from the content input unit 109. After the image processing unit 110 translates and rotates the content video in accordance with the displacement amount $(x, y, \theta)$ determined by the content video transmittance and displacement amount determining unit 108 (depending on a case, after the display size of the content video is further reduced in size), the image processing unit 110 outputs the video from the projector 111 (i.e., projects the video onto the transparent screen 112).

The transparent screen 112 is configured by, for example, laminating a film with electrically variable transmittance on a transparent film made of a resin or the like. The transparent screen 112 electrically controls the transmittance of the transparent film in accordance with the transmittance $\tau$ determined by the content video transmittance and displacement amount determining unit 108.

Configurations of the projector 111 and the transparent screen 112 (or arrangement positions in the vehicle) in a case where the display system is mounted on the vehicle have been described above in Item A. Moreover, the content video transmittance and displacement amount determining unit 108 determines the transmittance $\tau$ and the displacement amount $(x, y, \theta)$ of the projected video on the transparent screen 112 and the display size in accordance with the response level for motion sickness reduction. The point that adjusting the transmittance $\tau$ of the content video to be projected onto the transparent screen 112 and the movement and rotation $\theta$ of the display position $(x, y)$ and also the display size contributes to reduction of the motion sickness is as described above with reference to Figs. 16 to 23 in Item B.

Fig. 3 shows a processing procedure for the information processing system 100 to control the display of the content video in accordance with the motion sickness index of the occupant and the predicted displacement of the vehicle as a flowchart.

First of all, the occupant biometric information detection unit 101 detects biometric information such as heart beat rate and electroencephalography of the occupant (Step S301). Then, the motion sickness index computing unit 102 computes a motion sickness index of the occupant on the basis of the biometric information detected by the occupant biometric information detection unit 101 (Step S302).

Moreover, the velocity detection unit 103 detects velocity information of the vehicle, the acceleration detection unit 104 detects vehicle acceleration information, and the GPS information detection unit 105 analyzes a GPS signal received from a GPS satellite, measures the current location of the vehicle, and further detects map information of a driving planned route of the vehicle (Step S303). Then, the displacement prediction unit 106 computes a predicted displacement of the vehicle, i.e., a displacement amount of the vehicle after a certain time on the basis of the information detected by the respective detection units 103 to 105 (Step S304). It should be noted that the calculation processing of the motion sickness index of the occupant in Step S302 and the displacement prediction processing of the vehicle in Step S304 may be performed concurrently in parallel, not in chronological order.

Subsequently, the motion sickness reduction response level determining unit 107 determines a response level for motion sickness reduction from the determination table (see Fig. 2) determined in advance on the basis of the respective information, the motion sickness index computed by the motion sickness index computing unit 102 and the vehicle displacement amount predicted by the displacement prediction unit 106 (Step S305).

Subsequently, the content video transmittance and displacement amount determining unit 108 determines transmittance and a displacement amount of the content video that the projector 111 projects onto the transparent screen 112 on the basis of the response level determined by the motion sickness reduction response level determining unit 107 (Step S306). In Step S306, a display size of the content video may be further determined in addition to the transmittance and the displacement amount of the content video.

Then, the transparent screen 112 changes screen transmittance in accordance with the transmittance $\tau$ determined by the content video transmittance and displacement amount determining unit 108 (Step S307). Moreover, the image processing unit 110 translates and rotates the content video in accordance with the displacement amount $(x, y, \theta)$ and the display size determined by the content video transmittance and displacement amount determining unit 108 (depending on a case, after the display size of the content video is further reduced in size) (Step S308) and projects the content video onto the transparent screen 112 from the projector 111 (Step S309).

The processing procedure shown in Fig. 3 may be adapted to be executed by the information processing system 100 at the time of display start of the content video once or may be adapted to be executed repeatedly at predetermined time intervals during the display of the content video (or while the vehicle is moving).

The information processing system 100 controls the transmittance of the content video to be projected onto the transparent screen 112 in accordance with the motion sickness index of the occupant and the predicted displacement of the vehicle, such that the entire display can be used for recognizing the front. In this manner, it is possible to prevent or reduce the occurrence of the motion sickness of the occupant and reduce symptoms of the motion sickness of the occupant. The information processing system 100 may be adapted to control not only the transmittance of a region of the transparent screen 112, onto which the content video is projected, but also the transmittance of the entire transparent screen 112.

Moreover, the information processing system 100 controls the display position and rotation of the content video that the projector 111 projects onto the transparent screen 112 and also the display size in accordance with the motion sickness index of the occupant and the predicted displacement of the vehicle, such that visual sense information in front of the vehicle through the content video can be easily obtained. In this manner, it is possible to prevent or reduce the occurrence of the motion sickness of the occupant and reduce symptoms of the motion sickness.

D. System Configuration Example (2)

Fig. 4 shows a second configuration example of the information processing system 100. It should be noted that in Fig. 4, the same components as those shown in Fig. 1 are denoted by the same names and the same reference numerals and detailed descriptions thereof are omitted here.

The projector 111 is configured to project the content video onto the transparent screen 112 in the information processing system 100 shown in Fig. 1. On the other hand, the information processing system 100 is provided with a transparent FPD 113 as a display device in Fig. 4. That is a main difference.

After the image processing unit 110 translates and rotates a content video input from the content input unit 109 in accordance with the displacement amount $(x, y, \theta)$ determined by the content video transmittance and displacement amount determining unit 108 (depending on a case, after the display size of the content video is further reduced in size), the image processing unit 110 outputs it to the transparent FPD 113.

The transparent FPD 113 changes the transmittance of the entire transparent FPD 113 or a display region portion of the content video in accordance with the transmittance $\tau$ determined by the content video transmittance and displacement amount determining unit 108. The transparent FPD 113 is constituted by a transparent LCD, a transparent OLED, or another transparent display. The transparent display represented by the transparent LCD or the transparent OLED are well-known in the related art, so detailed descriptions thereof will be omitted in this specification.

The processing procedure executed by the information processing system 100 shown in Fig. 4 is similar to the flowchart shown in Fig. 3. It should be noted that it is assumed that in Step S307, the transparent FPD 113 changes the transmittance of the panel in accordance with the transmittance $\tau$ determined by the content video transmittance and displacement amount determining unit 108 and in Step S309, the transparent FPD 113 displays a video according to the displacement amount (x, y, $\theta$) and the display size determined by the content video transmittance and displacement amount determining unit 108.

Therefore, the information processing system 100 shown in Fig. 4 controls the transmittance of the transparent FPD 113 in accordance with the motion sickness index of the occupant and the predicted displacement of the vehicle, such that the entire display can be used for recognizing the front. In this manner, it is possible to prevent or reduce the occurrence of the motion sickness of the occupant and reduce symptoms of the motion sickness. Moreover, the information processing system 100 controls the position and rotation to display the content video on the transparent FPD 113 and also the display size in accordance with the motion sickness index of the occupant and the predicted displacement of the vehicle. In this manner, it is possible to prevent or reduce the occurrence of the motion sickness of the occupant and reduce symptoms of the motion sickness. The point that adjusting the transmittance $\tau$ of the content video and the movement and rotation $\theta$ of the display position (x, y) to be displayed on the transparent FPD 113 and also the display size contributes to reduction of the motion sickness is as described above with reference to Figs. 16 to 23 in Item B.

E. System Configuration Example (3)

Fig. 5 shows a third configuration example of the information processing system 100. It should be noted that in Fig. 5, the same components as those shown in Fig. 1 are denoted by the same names and the same reference numerals and detailed descriptions thereof are omitted here.

The information processing system 100 shown in Fig. 5 has main differences from the example shown in Fig. 1 in that the information processing system 100 further includes a background video capturing unit 114 and is provided with a non-transparent FPD 115 as a display device.

The background video capturing unit 114 is constituted by, for example, a vehicle-mounted camera and captures a video of a landscape in the periphery of the vehicle, for example, in front of the vehicle. The image processing unit 110 arranges a content video input from the content input unit 109 at the center, arranges the captured image received from the background video capturing unit 114 in the periphery, combines them into a single video, and outputs it to the non-transparent FPD 115. Therefore, the non-transparent FPD 115 displays an image obtained by superimposing the content video on a video see-through video.

Here, after the image processing unit 110 translates and rotates the content video input from the content input unit 109 in accordance with the displacement amount (x, y, $\theta$) determined by the content video transmittance and displacement amount determining unit 108 (depending on a case, after the display size of the content video is further reduced in size), the image processing unit 110 combines it on a background video input from the background video capturing unit 114. At this time, the image processing unit 110 adjusts a blend ratio of the content video to the background video in accordance with the transmittance $\tau$ determined by the content video transmittance and displacement amount determining unit 108. Then, the non-transparent FPD 115 displays an image obtained by the image processing unit 110 combining the content video on the captured image of the background video capturing unit 114 at the blend ratio corresponding to the transmittance $\tau$.

For example, the blend ratio of 1:0 is equivalent to the transmittance of 0% and the visibility of the content video is significantly high, but the field-of-view information in front of the vehicle is lost by an amount corresponding to the content video completely covering the background video. Moreover, the blend ratio of 0:1 is equivalent to the transmittance 100% and any content video cannot be visually recognized, but the field-of-view information in front of the vehicle increases correspondingly. In short, due to an increase in the blend ratio of the content video, the transmittance lowers and the visibility of the content video increases, but it becomes difficult to visually recognize the front of the vehicle. Moreover, due to a decrease in the blend ratio of the content video, the transmittance increases and the visibility of the content video lowers, but it becomes easy to visually recognize the front of the vehicle.

The processing procedure executed by the information processing system 100 shown in Fig. 5 is similar to the flowchart shown in Fig. 3. It should be noted that it is assumed that in Step S307, the blend ratio of the content video is determined in accordance with the transmittance $\tau$ determined by the content video transmittance and displacement amount determining unit 108. Moreover, it is assumed that in Step S308, the image processing unit 110 combines the content video obtained by translating and rotating the video (depending on a case, further reducing size of the display size of the content video) in accordance with the displacement amount (x, y, $\theta$) and the display size determined by the content video transmittance and displacement amount determining unit 108 on the background video captured by the background video capturing unit 114 at the blend ratio determined in Step S308. Then, it is assumed that in Step S309, the non-transparent FPD 115 displays the video see-through video combined by the image processing unit 110.

Therefore, the information processing system 100 shown in Fig. 5 controls the blend ratio of the content video to the video see-through video in accordance with the motion sickness index of the occupant and the predicted displacement of the vehicle, such that the entire display can be used for recognizing the front. In this manner, it is possible to prevent or reduce the occurrence of the motion sickness of the occupant and reduce symptoms of the motion sickness. Moreover, the information processing system 100 controls the position and rotation to superimpose the content video in the video see-through video in accordance with the motion sickness index of the occupant and the predicted displacement of the vehicle and also the display size. In this manner, it is possible to prevent or reduce the occurrence of the motion sickness of the occupant and reduce symptoms of the motion sickness. The point that adjusting the transmittance $\tau$ of the content video and the movement and rotation $\theta$ of the display position (x, y) displayed on the non-transparent FPD 115 and also the display size contributes to reduction of the motion sickness is as described above with reference to Figs. 16 to 23 in Item B.

F. System Configuration Example (4)

Fig. 6 shows a fourth configuration example of the information processing system 100. It should be noted that in Fig. 6, the same components as those shown in Fig. 1 are denoted by the same names and the same reference numerals and detailed descriptions thereof are omitted here.

The information processing system 100 shown in Fig. 6 is provided with a content video transmittance, displacement amount, and contrast determining unit 116 in place of the content video transmittance and displacement amount determining unit 108. This content video transmittance, displacement amount, and contrast determining unit 116 determines the contrast of the content video in addition to the transmittance $\tau$ and the displacement amount (x, y, $\theta$) of the content video that the projector 111 projects onto the transparent screen 112 on the basis of the response level determined by the motion sickness reduction response level determining unit 107.

After the image processing unit 110 translates and rotates the video and further adjusts the contrast in accordance with the displacement amount (x, y, $\theta$) determined by the content video transmittance, displacement amount, and contrast determining unit 116, the image processing unit 110 outputs the video from the projector 111 (i.e., projects the video onto the transparent screen 112).

The content video transmittance, displacement amount, and contrast determining unit 116 may be adapted to adjust the contrast of the video output from the projector 111 in accordance with the transmittance $\tau$. Then, the image processing unit 110 increases the contrast of the content video in a case where the transmittance $\tau$ is low and decreases the contrast of the content video in a case where the transmittance $\tau$ is high. As to a projected video with high contrast, the visibility increases while the transmittance decreases. On the other hand, as a projected video with low contrast, the transmittance increases by an amount corresponding a decrease in the visibility.

In accordance with the display system 100 shown in Fig. 6, the transparent screen 112 electrically controls the transmittance of the transparent film, such that the visibility of the content video and the field-of-view information in front of the vehicle can be adjusted by controlling the contrast of the content video to be projected from the projector 111 in addition to adjusting the transmittance $\tau$ of the content video.

The processing procedure executed by the information processing system 100 shown in Fig. 6 is similar to the flowchart shown in Fig. 3. It should be noted that it is assumed that in Step S308, the image processing unit 110 translates and rotates the content video in accordance with the displacement amount (x, y, $\theta$) and the display size determined by the content video transmittance, displacement amount, and contrast determining unit 116 and also sets the contrast of the content video determined by the content video transmittance, displacement amount, and contrast determining unit 116, and in Step S309, the image processing unit 110 projects the content video with the contrast determined by the content video transmittance, displacement amount, and contrast determining unit 116 onto the transparent screen 112 from the projector 111.

Therefore, the information processing system 100 shown in Fig. 6 controls the transmittance and contrast of the content video in accordance with the motion sickness index of the occupant and the predicted displacement of the vehicle, such that the entire display can be used for recognizing the front. In this manner, it is possible to prevent or reduce the occurrence of the motion sickness of the occupant and reduce symptoms of the motion sickness. Moreover, the information processing system 100 controls the position and rotation to display the content video in accordance with the motion sickness index of the occupant and the predicted displacement of the vehicle. In this manner, it is possible to prevent or reduce the occurrence of the motion sickness of the occupant and reduce symptoms of the motion sickness. The point that adjusting the transmittance $\tau$ of the content video and the movement and rotation $\theta$ of the display position (x, y) to be displayed on the transparent screen 112 and also the display size contributes to reduction of the motion sickness should be understood from the above description with reference to Figs. 16 to 23 in Item B.

G. System Configuration Example (5)

Fig. 7 shows a fifth configuration example of the information processing system 100. It should be noted that in Fig.

7, the same components as those shown in Fig. 1 are denoted by the same names and the same reference numerals and detailed descriptions thereof are omitted here.

The information processing system 100 shown in Fig. 7 is mainly characterized in that the information processing system 100 includes a head motion computing unit 701, a motion sickness accumulation factor computing unit 702, a motion sickness recovery factor computing unit 703, and a motion sickness index computing unit 704 in place of the occupant biometric information detection unit 101 and the motion sickness index computing unit 102.

The head motion computing unit 701 computes a head motion of the occupant on the basis of respective data detected by the in-vehicle sensors. Basically, the head motion computing unit 701 calculates a head motion of the occupant by simulation calculation on the basis of the motion of the vehicle. Specifically, the head motion computing unit 701 calculates head acceleration ($A_X$, $A_y$, $A_z$) of the occupant by simulation calculation using a simulation model of the human body (upper half of the body) on the basis of vehicle acceleration ($a_x$, $a_y$, $a_z$) detected by the acceleration sensor mounted on a rigid part of the vehicle and attribute data of the occupant (a sitting height (h) and a weight (w) of the upper half of the body) detected from the in-vehicle monitoring camera and the body pressure sensor mounted on the seat surface. It should be noted that the head motion computing unit 701 may be adapted to detect an actual head motion from the captured image of the occupant or estimate a head motion on the basis of the vehicle's driving route and a motion in a camera video other than the above-mentioned simulation calculation.

The motion sickness accumulation factor computing unit 702 performs weighting on the head acceleration ($A_x$, $A_y$, $A_z$) calculated by the head motion computing unit 701 in accordance with a frequency, detects a traveling time in which the vehicle is driven, and calculates an accumulation factor of motion sickness of the occupant by time integration using Expression described in ISO 2631.

The motion sickness recovery factor computing unit 703 calculates a recovery factor of the motion sickness of the occupant on the basis of the detected attribute data (ditto) of the occupant and the detected traveling time of the vehicle. Moreover, the motion sickness recovery factor computing unit 703 may calculate the recovery factor of the motion sickness of the occupant in consideration of the position of the occupant in the vehicle and the posture of the occupant in addition to the detected attribute data (ditto) of the occupant and the detected traveling time of the vehicle. Even in the same vehicle, the motion sickness recovery degree changes depending on the occupant's sitting position. For example, in a case of a vehicle in which seats of three or more rows are installed, the front field-of-view and up and down, left and right motions are not uniform for each seat position, and the head motion varies depending on the occupant's sitting position. For example, on the basis of the image captured by the in-vehicle monitoring camera, the position of the occupant in the vehicle and the posture of the occupant are acquired.

The motion sickness index computing unit 704 combines the accumulation factor of the motion sickness calculated by the motion sickness accumulation factor computing unit 702 and the recovery factor of the motion sickness calculated by the motion sickness recovery factor computing unit 703 to calculate a motion sickness index of the occupant.

The motion sickness reduction response level determining unit 107 determines a response level for motion sickness reduction on the basis of respective information of the motion sickness of the occupant, which has been computed by the motion sickness index computing unit 704 in consideration of the accumulation factor of the motion sickness and the recovery factor of the motion sickness, and the displacement amount predicted by the displacement prediction unit 106.

The content video transmittance and displacement amount determining unit 108 determines the transmittance and the displacement amount of the content video that the projector 111 projects onto the transparent screen 112 on the basis of the response level determined by the motion sickness reduction response level determining unit 107.

A video signal of the content that the projector 111 projects onto the transparent screen 112 is input to the image processing unit 110 from the content input unit 109. After the image processing unit 110 translates and rotates the video in accordance with the displacement amount determined by the content video transmittance and displacement amount determining unit 108, the image processing unit 110 outputs the video from the projector 111. Moreover, the transparent screen 112 changes the screen transmittance in accordance with the transmittance determined by the content video transmittance and displacement amount determining unit 108.

As to the motion sickness, ISO 2631 has clearly described that it can be calculated from a behavior (acceleration) of the occupant's head. The motion sickness dose value (MSDV) according to ISO 2631 is an index of the motion sickness, which is calculated by time integration of the head motion, in other words, an accumulation factor of motion sickness.

Specifically, the motion sickness accumulation factor computing unit 702 calculates a determination index of the motion sickness (motion sickness dose value) by time integration using Expression (1) below described in ISO 2631.

[Formula 1]

$$MSDV = \sqrt{\int_0^T [a_W(t)]^2 dt} \quad \cdots (1)$$

It should be noted that in Expression (1) above, $a_w(t)$ is weighted effective acceleration (weighted RMS acceleration) of the vehicle at a time t and is acceleration obtained by weighting vehicle acceleration a(t), which depends on a frequency. The weighted effective acceleration $a_w(t)$ can be obtained by converting the acceleration a(t) of a time domain into a frequency domain once, multiplying it by a transfer function H(s) of the filter (it should be noted that $s = j\omega = j2\pi f$), and then converting it back to the time domain. The correlation coefficient $\alpha$ between the determination index MSDV of the motion sickness and a simulator sickness questionnaire (SSQ) as proneness to the motion sickness can be determined by experiments. The motion sickness accumulation factor computing unit 702 calculates an accumulation factor $\alpha$*MSDV of the motion sickness of the occupant by using this correlation coefficient $\alpha$.

However, it has been confirmed by experimental evaluation that symptoms of the motion sickness differ depending on how the boarded vehicle is traveling even in a case where the time-integral value of the head motion is the same. It can be considered from this experimental evaluation that the occupant tends to recover from the motion sickness when there is a less head motion during a vehicle boarding time. In view of this, in the information processing system 100 shown in Fig. 7, the motion sickness recovery factor computing unit 703 further calculates a recovery factor R of the motion sickness of the occupant on the basis of the attribute data (ditto) of the occupant and the traveling time of the vehicle. Then, the motion sickness index computing unit 704 is configured to combine the accumulation factor $\alpha$*MSDV of the motion sickness and the recovery factor R of the motion sickness to more accurately calculate a motion sickness index S (= $\alpha$*MSDV - R).

Moreover, in the information processing system 100 shown in Fig. 7, in consideration of the fact that a method of directly detecting a head motion is difficult and the accuracy of detection is also poor, the head motion of the occupant is calculated by simulation on the basis of the motion of the vehicle. Using the in-vehicle sensors such as the acceleration sensor, behaviors of the vehicle can be precisely detected. The head motion can be calculated by simulation prediction further on the basis of body information such as a weight and a sitting height of the occupant.

The processing procedure executed by the information processing system 100 shown in Fig. 7 is similar to the flowchart shown in Fig. 3. It should be noted that it is assumed that in Step S301, the head motion computing unit 701 calculates head acceleration of the occupant by simulation calculation using the simulation model of the human body (upper half of the body) on the basis of the vehicle acceleration and the attribute data of the occupant (the sitting height (h) and the weight (w) of the upper half of the body), and in Step S302, the motion sickness accumulation factor computing unit 702 integrally calculates an accumulation factor of motion sickness of the occupant on the basis of the head motion of the occupant obtained by simulation calculation and the motion sickness recovery factor computing unit 703 calculates a recovery factor of the motion sickness, and the motion sickness index computing unit 704 combines the accumulation factor and the recovery factor of the motion sickness to calculate a motion sickness index.

Therefore, the information processing system 100 shown in Fig. 7 controls the transmittance, the display position and rotation of the content video that the projector 111 projects the content video onto the transparent screen 112 and also the display size in accordance with the motion sickness index of the occupant and the predicted displacement of the vehicle, such that visual sense information in front of the vehicle through the content video can be easily obtained. In this manner, it is possible to prevent or reduce the occurrence of the motion sickness of the occupant and reduce symptoms of the motion sickness. Moreover, the information processing system 100 more correctly calculates a motion sickness index of the occupant in consideration of the accumulation factor and the recovery factor of the motion sickness, and therefore the transmittance of the projected video is actually not unnecessarily reduced when the occupant does not suffer from motion sickness, and the visibility of the content video can be kept.

It should be noted that the computing of the motion sickness based on the simulation prediction of the head motion (accumulation factor of the motion sickness) as shown in Fig. 7 and the computing of the motion sickness index considering the accumulation factor and the recovery factor of the motion sickness can also be applied to the information processing system using another type of display system as shown in Fig. 1 and Figs. 4 to 6.

Industrial Applicability

Hereinabove, the present disclosure has been described in detail with reference to specific embodiments. However, it is obvious that those skilled in the art may make modifications or substitutions to the embodiments without departing from the gist of the present disclosure.

Although this description has focused on the embodiments in which the present disclosure is applied to the reduction of the motion sickness while the occupant is in the vehicle, the gist of the present disclosure is not limited thereto. The present disclosure can also be applied to motion sickness caused by motion of any one of various vehicles (movable objects) including a boat and an airplane when the occupant who has boarded the vehicle (movable object) views a content video in the vehicle (movable object).

In short, the present disclosure has been described by way of example and should not be construed as limiting the contents of the present disclosure. The scope of claims should be considered to determine the gist of the present disclosure.

It should be noted that the present disclosure can also take configurations as follows.

(1) An information processing apparatus, including:

a determining unit that determines motion sickness of an occupant in a movable object; and
a processing unit that performs, on the basis of a determination result of the determining unit, processing for determining transmittance of at least a partial region with a transmittance variable region in a display system that is mounted on the movable object and displays a video in the transmittance variable region.

(2) The information processing apparatus according to (1), in which
the determining unit determines the motion sickness on the basis of at least one of a motion sickness index of the occupant or a predicted displacement of the movable object.
(3) The information processing apparatus according to any one of (1) or (2), in which
the determining unit determines the motion sickness of the occupant on the basis of biometric information of the occupant.
(4) The information processing apparatus according to any one of (1) to (3), in which
the determining unit determines the motion sickness of the occupant on the basis of an accumulation factor and a recovery factor of the motion sickness.
(5) The information processing apparatus according to (4), in which
the determining unit computes the accumulation factor of the motion sickness on the basis of time integration of a head motion of the occupant.
(6) The information processing apparatus according to any one of (4) or (5), in which
the determining unit computes the accumulation factor of the motion sickness on the basis of time integration of a head motion of the occupant, which is predicted by head motion simulation computing based on acceleration of the movable object and body information of the occupant.
(7) The information processing apparatus according to any one of (4) to (6), in which
the determining unit computes the recovery factor of the motion sickness on the basis of a traveling time of the movable object and body information of the occupant.
(8) The information processing apparatus according to any one of (1) to (7), in which
the determining unit predicts a displacement of the movable object on the basis of at least one of velocity information, acceleration information, or positional information of the movable object.
(9) The information processing apparatus according to any one of (1) to (8), in which

the determining unit determines a response level of the motion sickness on the basis of a combination of a motion sickness index of the occupant and a predicted displacement of the movable object, and
the processing unit determines transmittance of at least a partial region of the transmittance variable region on the basis of the response level of the motion sickness which is determined by the determining unit.

(10) The information processing apparatus according to any one of (1) to (9), in which
the processing unit determines transmittance of a video to be displayed in the transmittance variable region.
(11) The information processing apparatus according to any one of (1) to (10), in which
the processing unit further determines a displacement amount of at least one of a display position or an angle of a video in the transmittance variable region.
(12) The information processing apparatus according to any one of (1) to (11), in which

the display system includes a transparent screen with variable transmittance and a projector that projects a video on the transparent screen, and
the processing unit determines transmittance of the entire transparent screen or transmittance of the video to be projected on the transparent screen.

(13) The information processing apparatus according to any one of (1) to (11), in which

the display system includes a transparent flat panel display with variable transmittance, and
the processing unit determines transmittance of the entire transparent flat panel display or transmittance of a video display region on the transparent flat panel display.

(14) The information processing apparatus according to any one of (1) to (11), in which

the display system includes an imaging unit that images a periphery of the movable object and a non-transparent flat panel display, and
the processing unit combines a captured video and a content video of the imaging unit at a blend ratio based on the determined transmittance and displays the combined captured video and content video on the non-transparent flat panel display.

(15) The information processing apparatus according to any one of (1) to (11), in which
the processing unit further determines a contrast of the video to be displayed in the transmittance variable region.
(16) An information processing method, including:

a determining step of determining motion sickness of an occupant in a movable object; and
a processing step of performing, on the basis of a determination result of the determining step, processing for determining transmittance of at least a partial region with a transmittance variable region in a display system that is mounted on the movable object and displays a video in the transmittance variable region.

(17) A system, including:

a display system that is mounted on a movable object and displays a video in a transmittance variable region;
a determining unit that determines motion sickness of an occupant in the movable object; and
a processing unit that performs, on the basis of a determination result of the determining unit, processing for determining transmittance of at least a partial region with a transmittance variable region in the display system.

Reference Signs List

100 information processing system
101 occupant biometric information detection unit
102 motion sickness index computing unit
103 velocity detection unit
104 acceleration detection unit
105 GPS information detection unit
106 displacement prediction unit
107 motion sickness reduction response level determining unit
108 content video transmittance and displacement amount determining unit
109 content input unit
110 image processing unit
111 projector
112 transparent screen
113 transparent FPD
114 background video capturing unit
115 non-transparent FPD
116 content video transmittance, displacement amount, and contrast determining unit
701 head motion computing unit
702 motion sickness accumulation factor computing unit
703 motion sickness recovery factor computing unit
704 motion sickness index computing unit

## Claims

**1.** An information processing apparatus, comprising:

a determining unit that determines motion sickness of an occupant in a movable object; and
a processing unit that performs, on a basis of a determination result of the determining unit, processing for determining transmittance of at least a partial region with a transmittance variable region in a display system that is mounted on the movable object and displays a video in the transmittance variable region.

2. The information processing apparatus according to claim 1, wherein the determining unit determines the motion sickness on a basis of at least one of a motion sickness index of the occupant or a predicted displacement of the movable object.

3. The information processing apparatus according to claim 1, wherein the determining unit determines the motion sickness of the occupant on a basis of biometric information of the occupant.

4. The information processing apparatus according to claim 1, wherein the determining unit determines the motion sickness of the occupant on a basis of an accumulation factor and a recovery factor of the motion sickness.

5. The information processing apparatus according to claim 4, wherein the determining unit computes the accumulation factor of the motion sickness on a basis of time integration of a head motion of the occupant.

6. The information processing apparatus according to claim 4, wherein the determining unit computes the accumulation factor of the motion sickness on a basis of time integration of a head motion of the occupant, which is predicted by head motion simulation computing based on acceleration of the movable object and body information of the occupant.

7. The information processing apparatus according to claim 4, wherein the determining unit computes the recovery factor of the motion sickness on a basis of a traveling time of the movable object and body information of the occupant.

8. The information processing apparatus according to claim 1, wherein the determining unit predicts a displacement of the movable object on a basis of at least one of velocity information, acceleration information, or positional information of the movable object.

9. The information processing apparatus according to claim 1, wherein

the determining unit determines a response level of the motion sickness on a basis of a combination of a motion sickness index of the occupant and a predicted displacement of the movable object, and
the processing unit determines transmittance of at least a partial region of the transmittance variable region on a basis of the response level of the motion sickness which is determined by the determining unit.

10. The information processing apparatus according to claim 1, wherein the processing unit determines transmittance of a video to be displayed in the transmittance variable region.

11. The information processing apparatus according to claim 1, wherein the processing unit further determines a displacement amount of at least one of a display position or an angle of a video in the transmittance variable region.

12. The information processing apparatus according to claim 1, wherein

the display system includes a transparent screen with variable transmittance and a projector that projects a video on the transparent screen, and
the processing unit determines transmittance of the entire transparent screen or transmittance of the video to be projected on the transparent screen.

13. The information processing apparatus according to claim 1, wherein

the display system includes a transparent flat panel display with variable transmittance, and
the processing unit determines transmittance of the entire transparent flat panel display or transmittance of a video display region on the transparent flat panel display.

14. The information processing apparatus according to claim 1, wherein

the display system includes an imaging unit that images a periphery of the movable object and a non-transparent flat panel display, and
the processing unit combines a captured video and a content video of the imaging unit at a blend ratio based on the determined transmittance and displays the combined captured video and content video on the non-transparent flat panel display.

**15.** The information processing apparatus according to claim 1, wherein
the processing unit further determines a contrast of the video to be displayed in the transmittance variable region.

**16.** An information processing method, comprising:

a determining step of determining motion sickness of an occupant in a movable object; and
a processing step of performing, on a basis of a determination result of the determining step, processing for determining transmittance of at least a partial region with a transmittance variable region in a display system that is mounted on the movable object and displays a video in the transmittance variable region.

**17.** A system, comprising:

a display system that is mounted on a movable object and displays a video in a transmittance variable region;
a determining unit that determines motion sickness of an occupant in the movable object; and
a processing unit that performs, on a basis of a determination result of the determining unit, processing for determining transmittance of at least a partial region with a transmittance variable region in the display system.

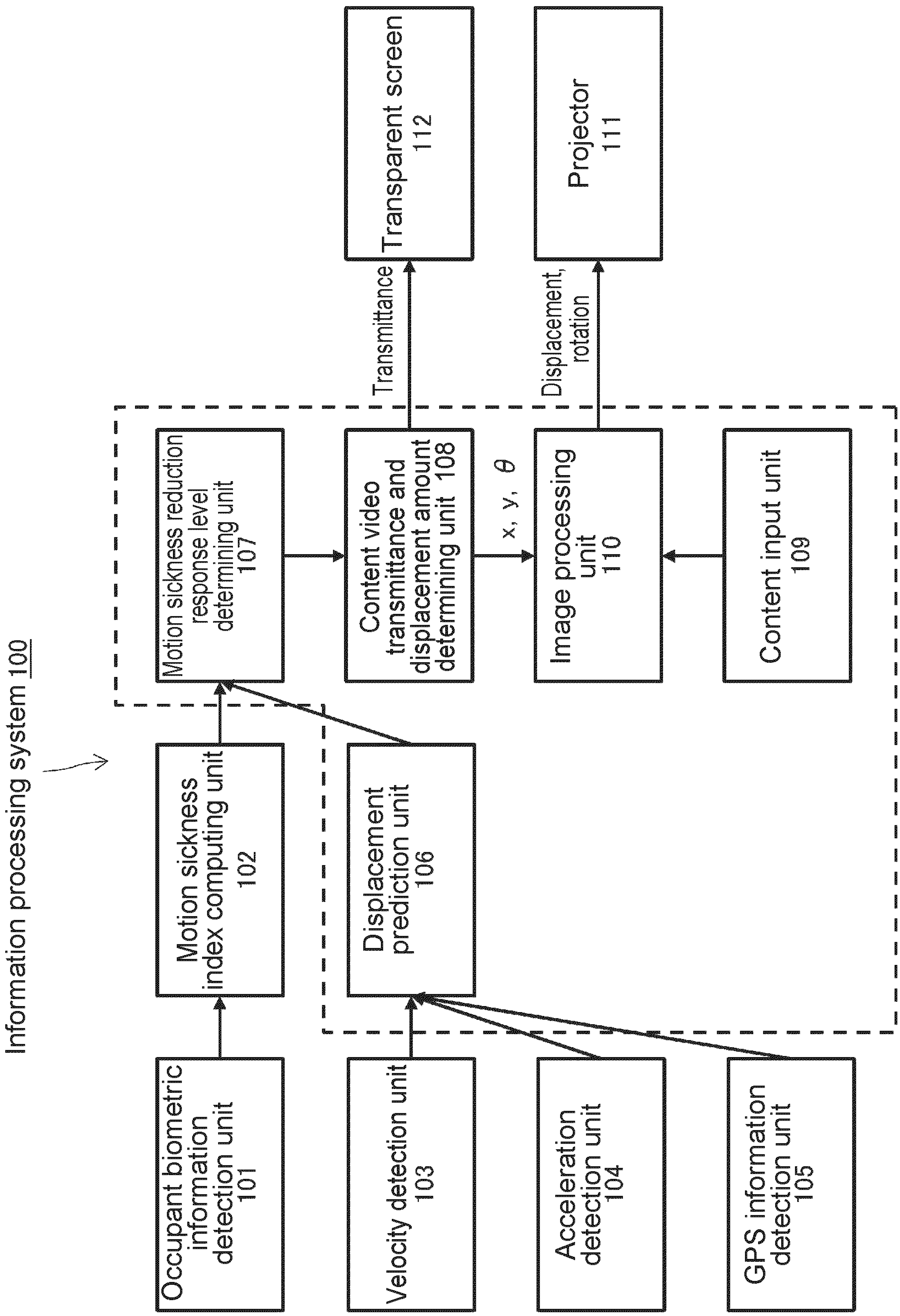
Information processing system 100
Occupant biometric information detection unit 101
Velocity detection unit 103
Acceleration detection unit 104
GPS information detection unit 105
Motion sickness index computing unit 102
Displacement prediction unit 106
Motion sickness reduction response level determining unit 107
Content video transmittance and displacement amount determining unit 108
x, y, θ
Image processing unit 110
Content input unit 109
Transmittance
Transparent screen 112
Displacement, rotation
Projector 111

FIG.1

| Response level for motion sickness reduction | | Motion sickness index | | |
|---|---|---|---|---|
| | | Small | Medium | Large |
| Predicted displacement amount | Small | 10% | 30% | 50% |
| | Medium | 30% | 50% | 70% |
| | Large | 50% | 70% | 90% |

FIG.2

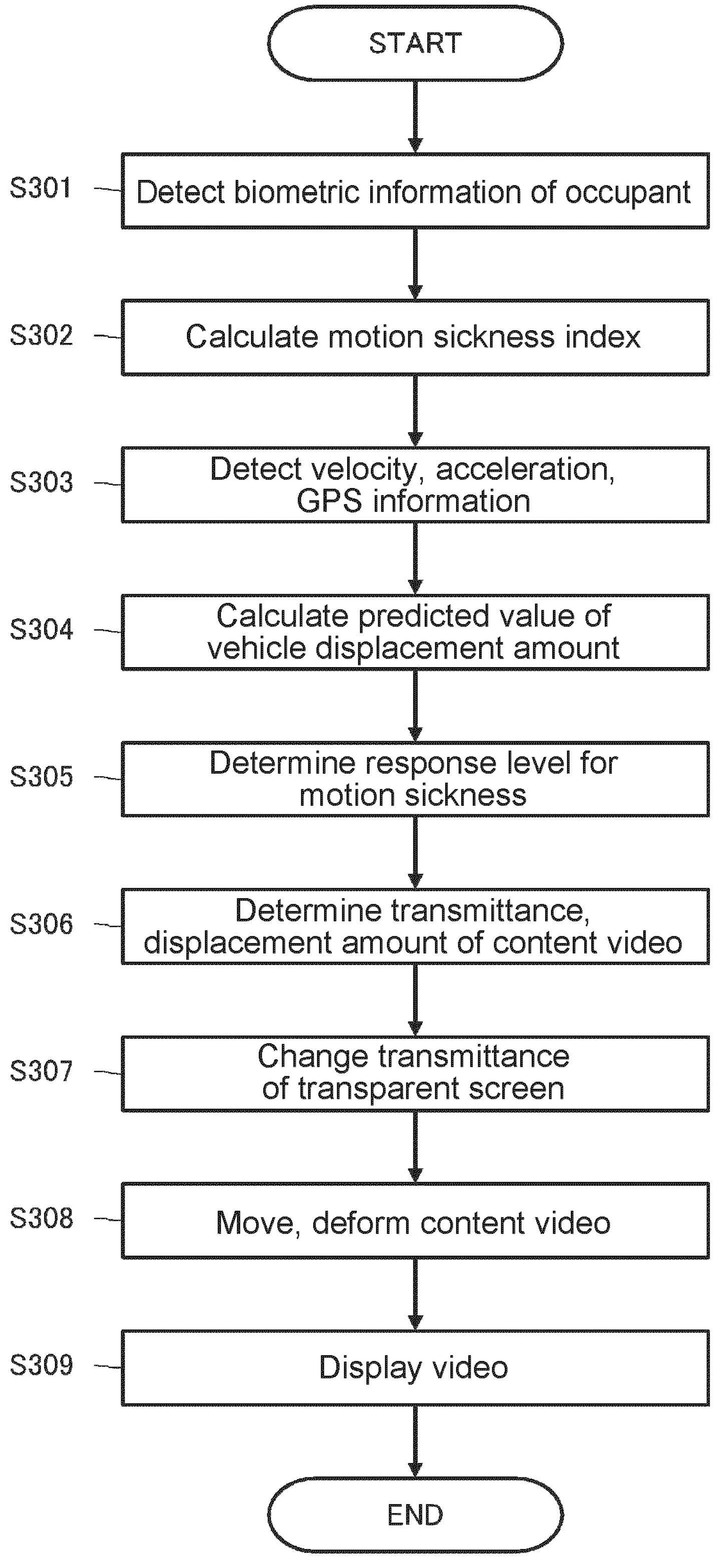
START
S301
Detect biometric information of occupant
S302
Calculate motion sickness index
S303
Detect velocity, acceleration, GPS information
S304
Calculate predicted value of vehicle displacement amount
S305
Determine response level for motion sickness
S306
Determine transmittance, displacement amount of content video
S307
Change transmittance of transparent screen
S308
Move, deform content video
S309
Display video
END

FIG.3

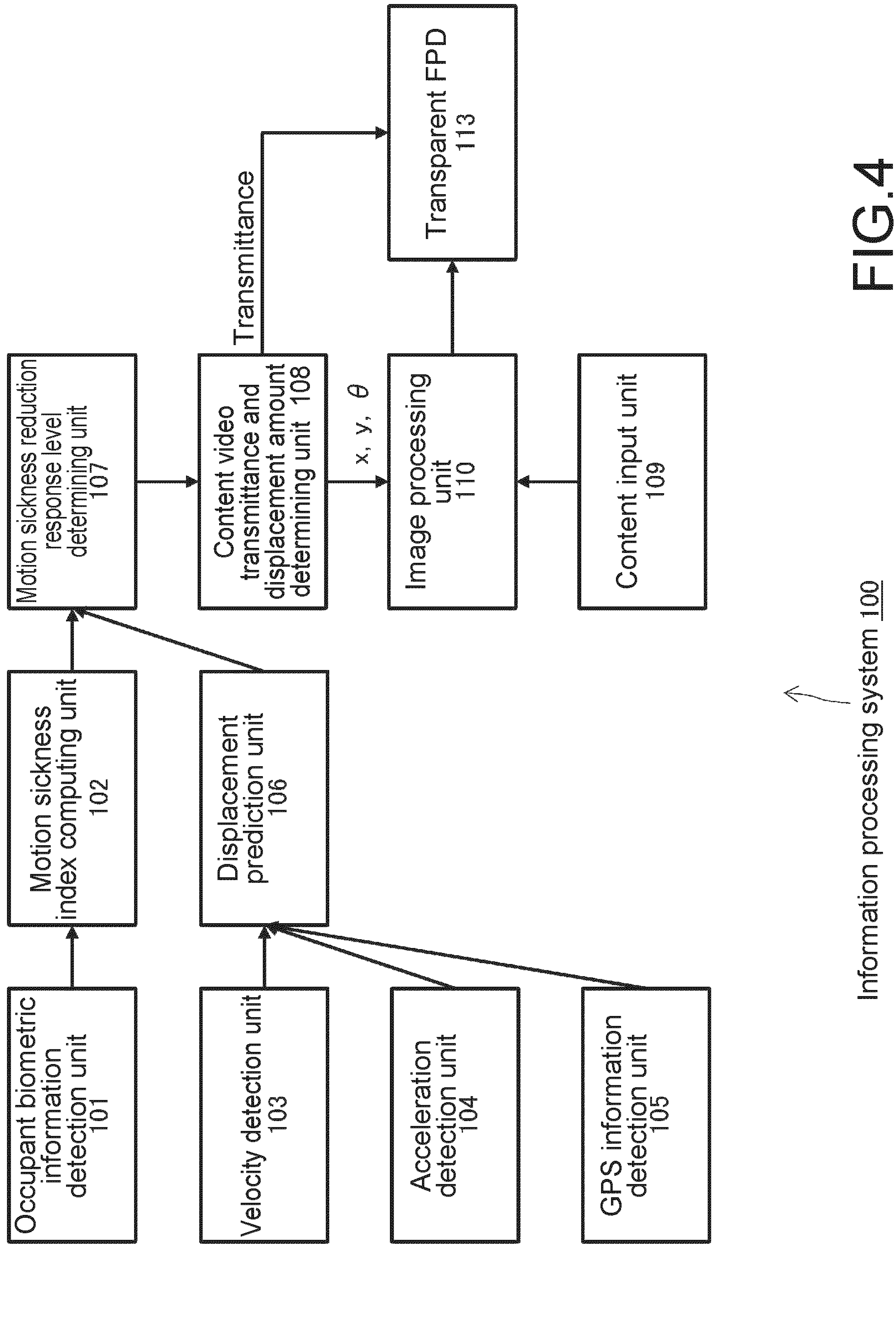
Occupant biometric information detection unit 101
Velocity detection unit 103
Acceleration detection unit 104
GPS information detection unit 105
Motion sickness index computing unit 102
Displacement prediction unit 106
Motion sickness reduction response level determining unit 107
Content video transmittance and displacement amount determining unit 108
Transmittance
x, y, θ
Image processing unit 110
Content input unit 109
Transparent FPD 113
Information processing system 100

FIG.4

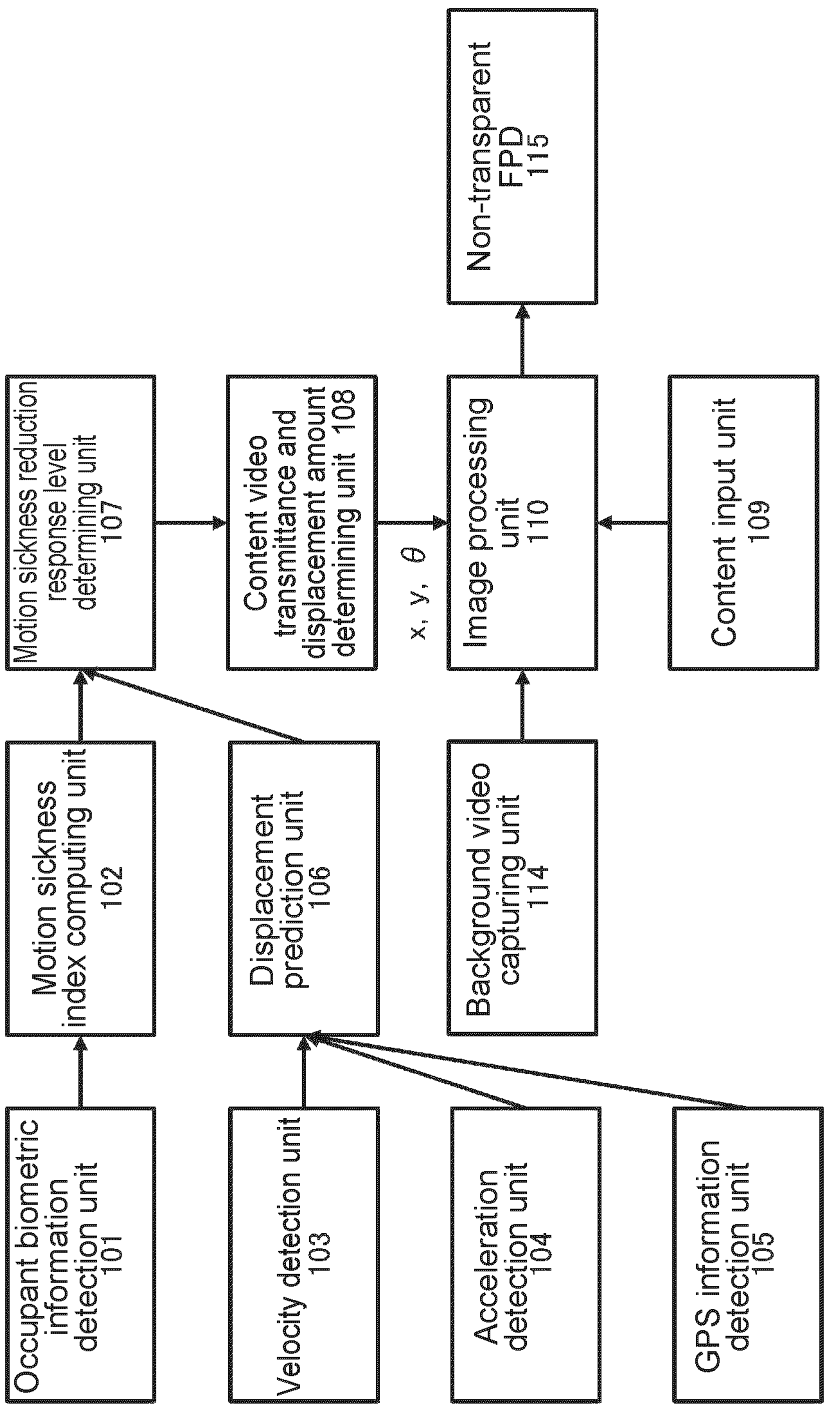


Information processing system 100

FIG.5

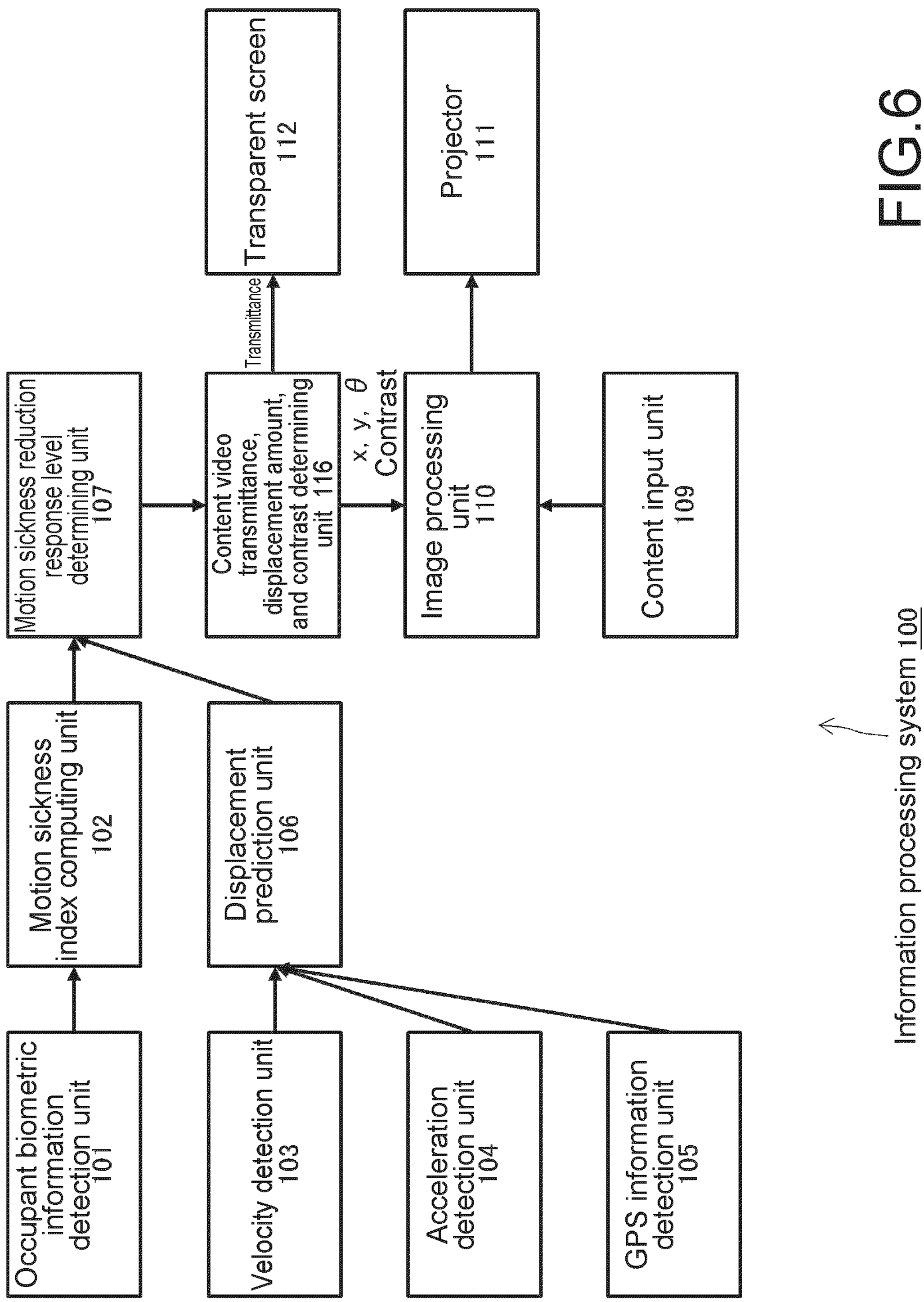


Information processing system 100

FIG.6

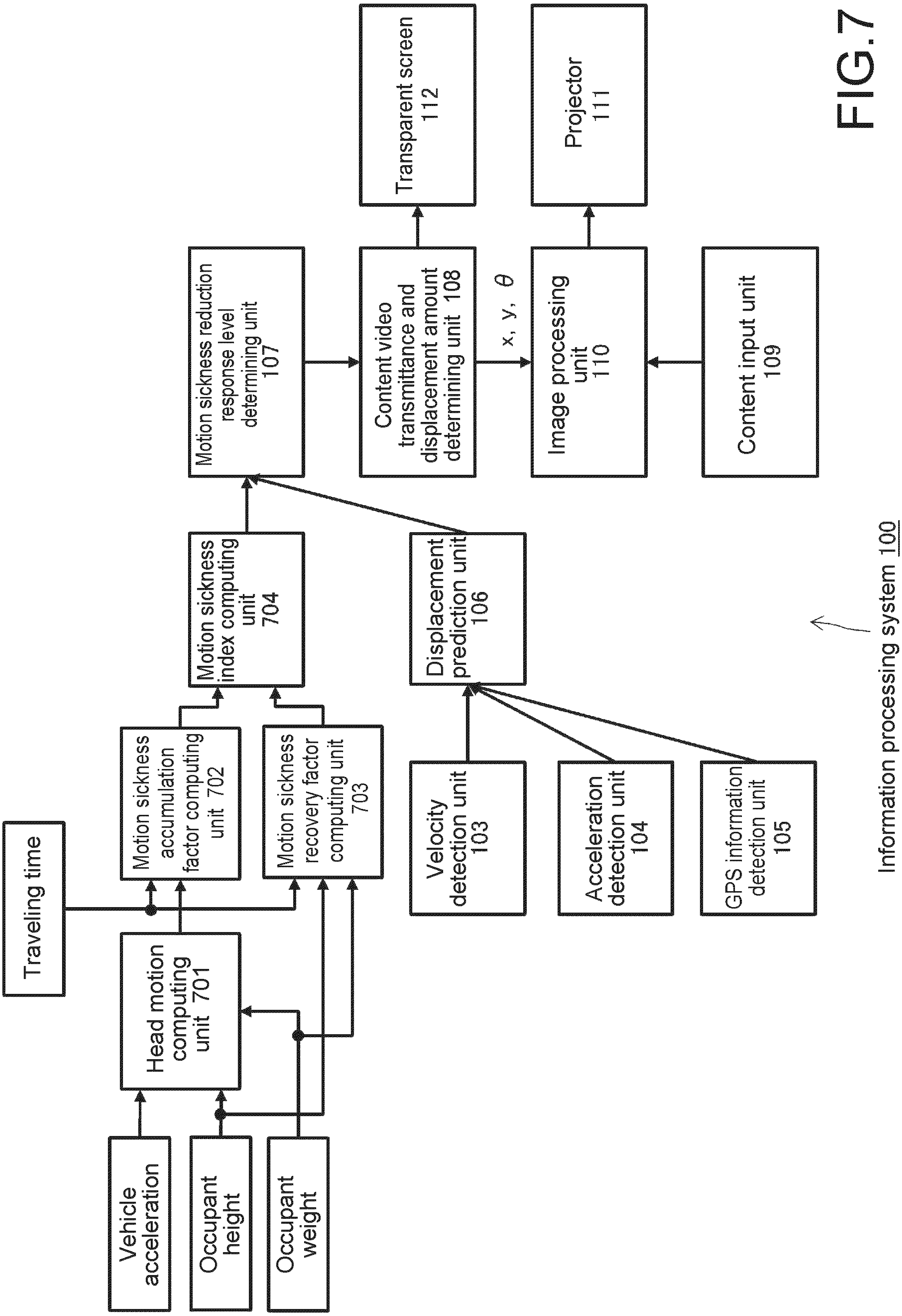
Traveling time
Vehicle acceleration
Occupant height
Occupant weight
Head motion computing unit 701
Motion sickness accumulation factor computing unit 702
Motion sickness recovery factor computing unit 703
Motion sickness index computing unit 704
Velocity detection unit 103
Acceleration detection unit 104
GPS information detection unit 105
Displacement prediction unit 106
Motion sickness reduction response level determining unit 107
Content video transmittance and displacement amount determining unit 108
x, y, θ
Image processing unit 110
Content input unit 109
Transparent screen 112
Projector 111
Information processing system 100

FIG.7

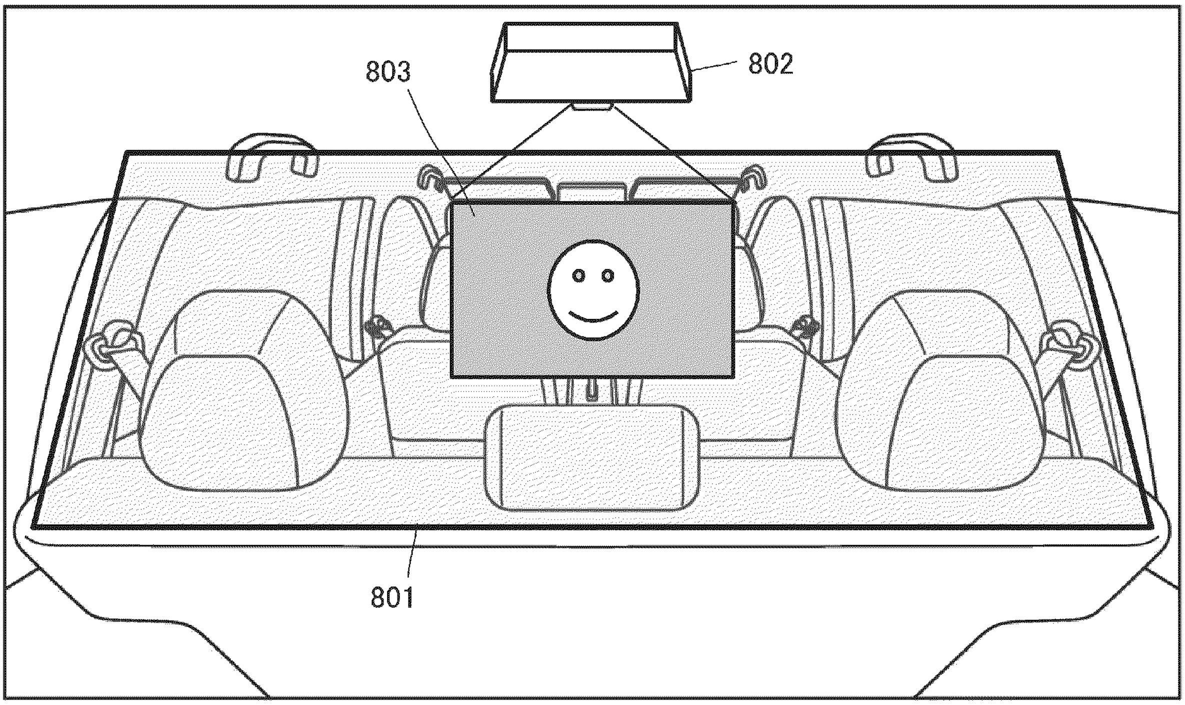


FIG.8

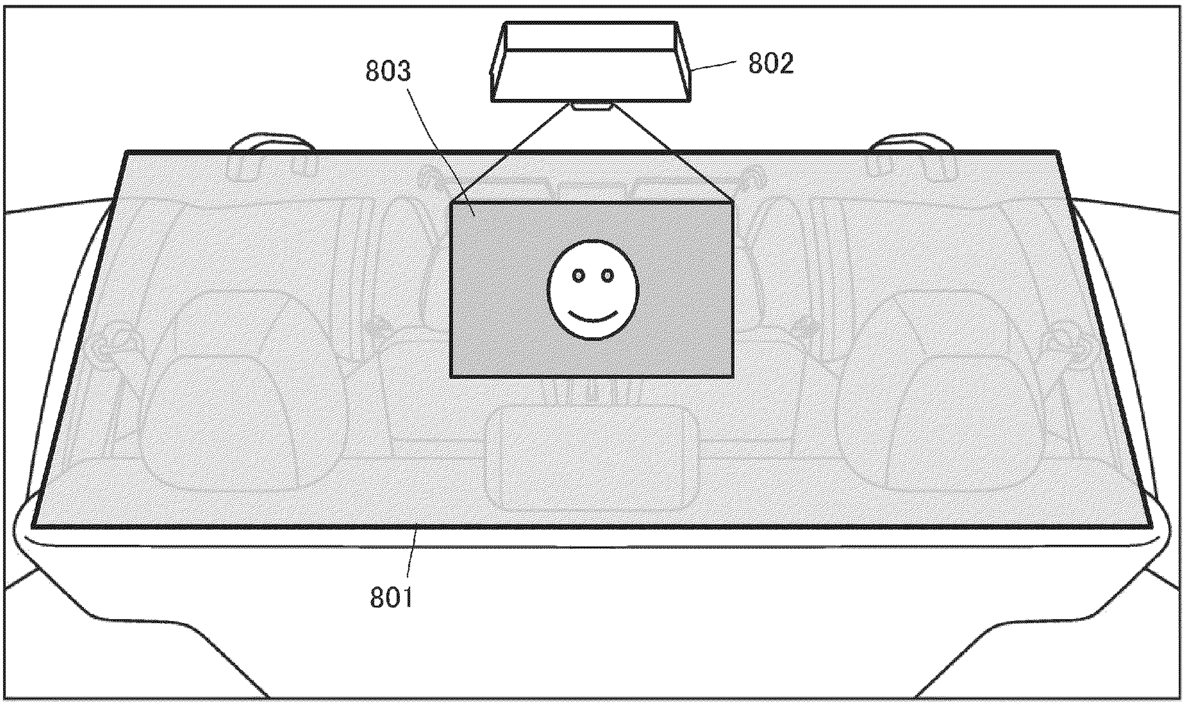


FIG.9

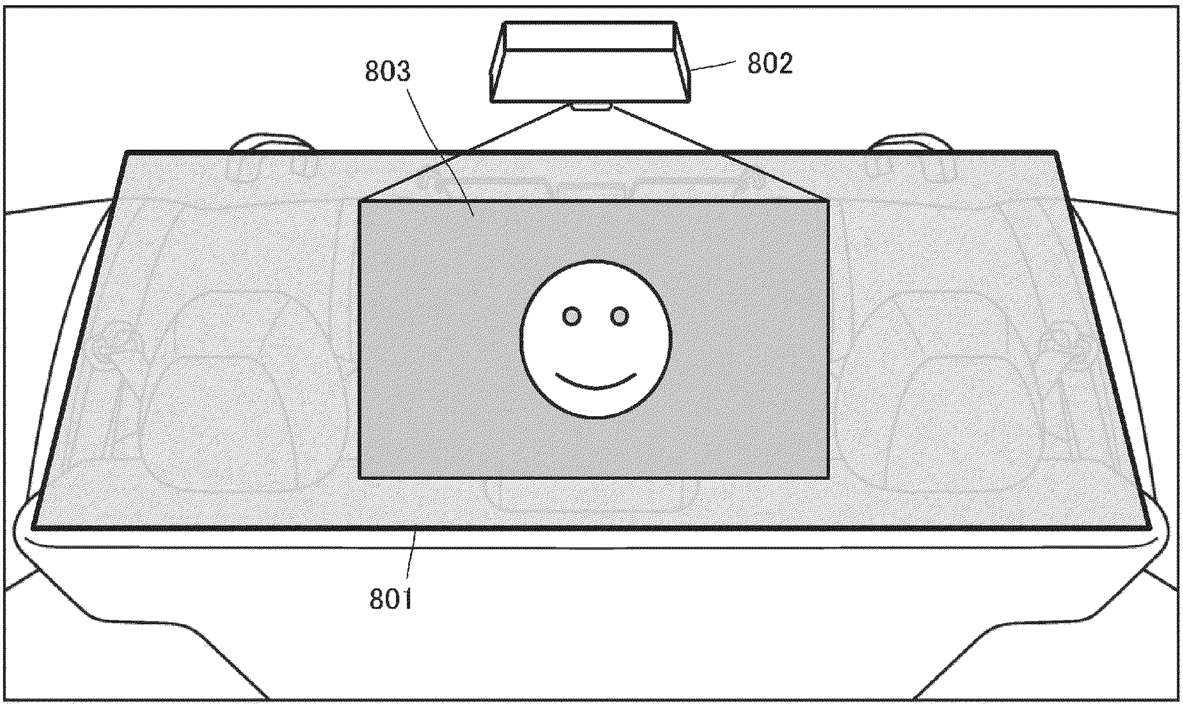


FIG.10

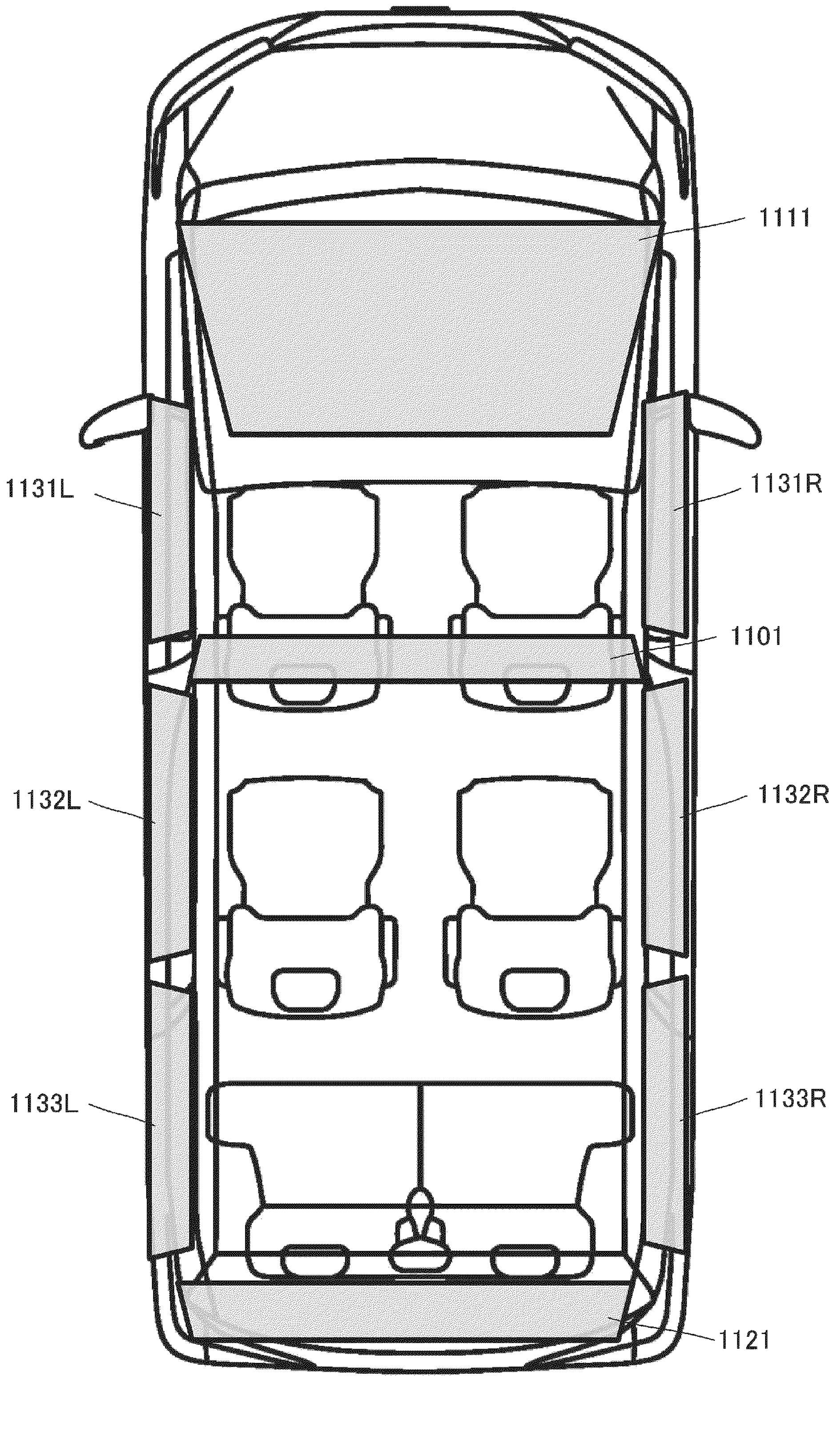


FIG.11

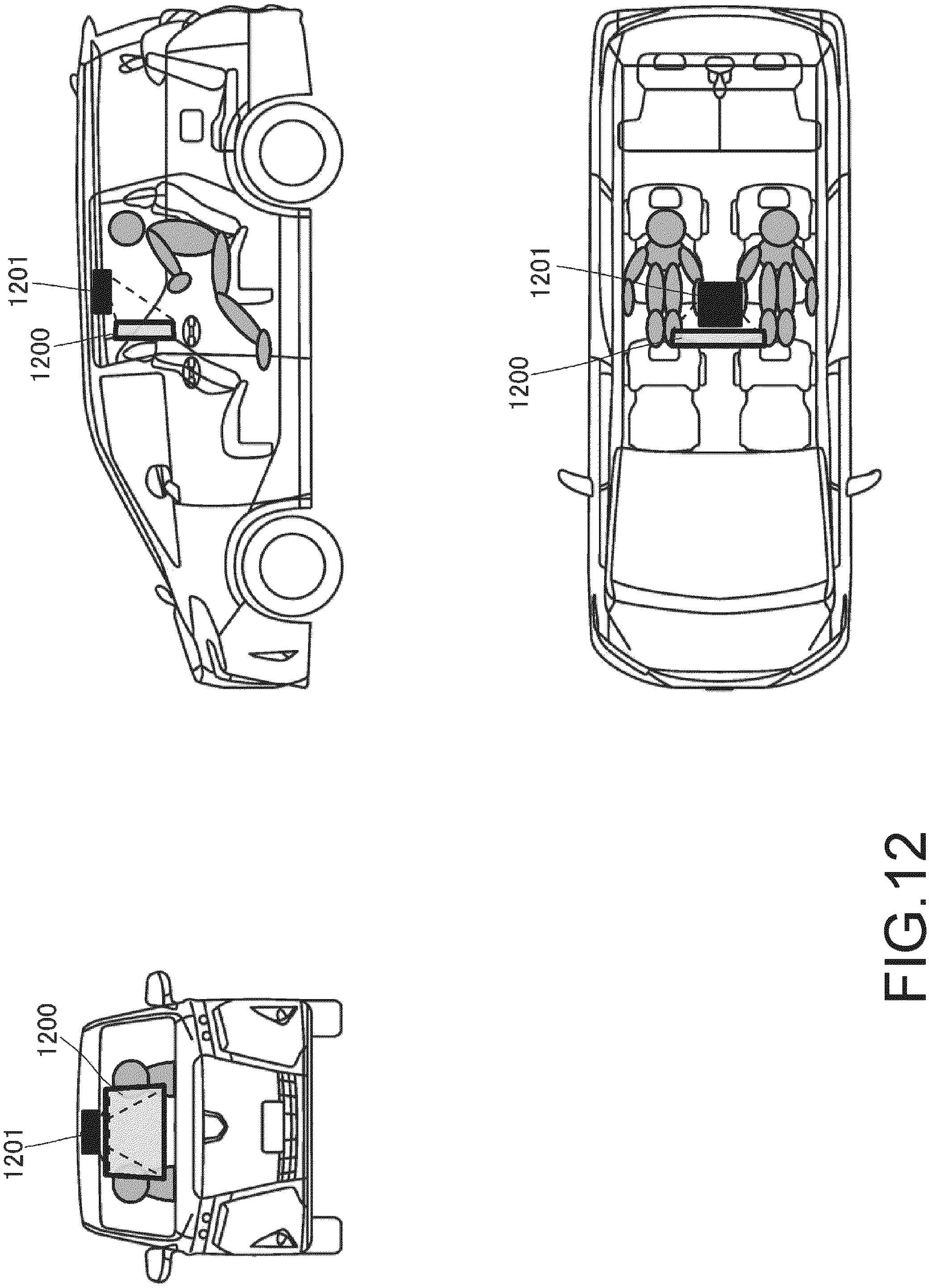


FIG.12

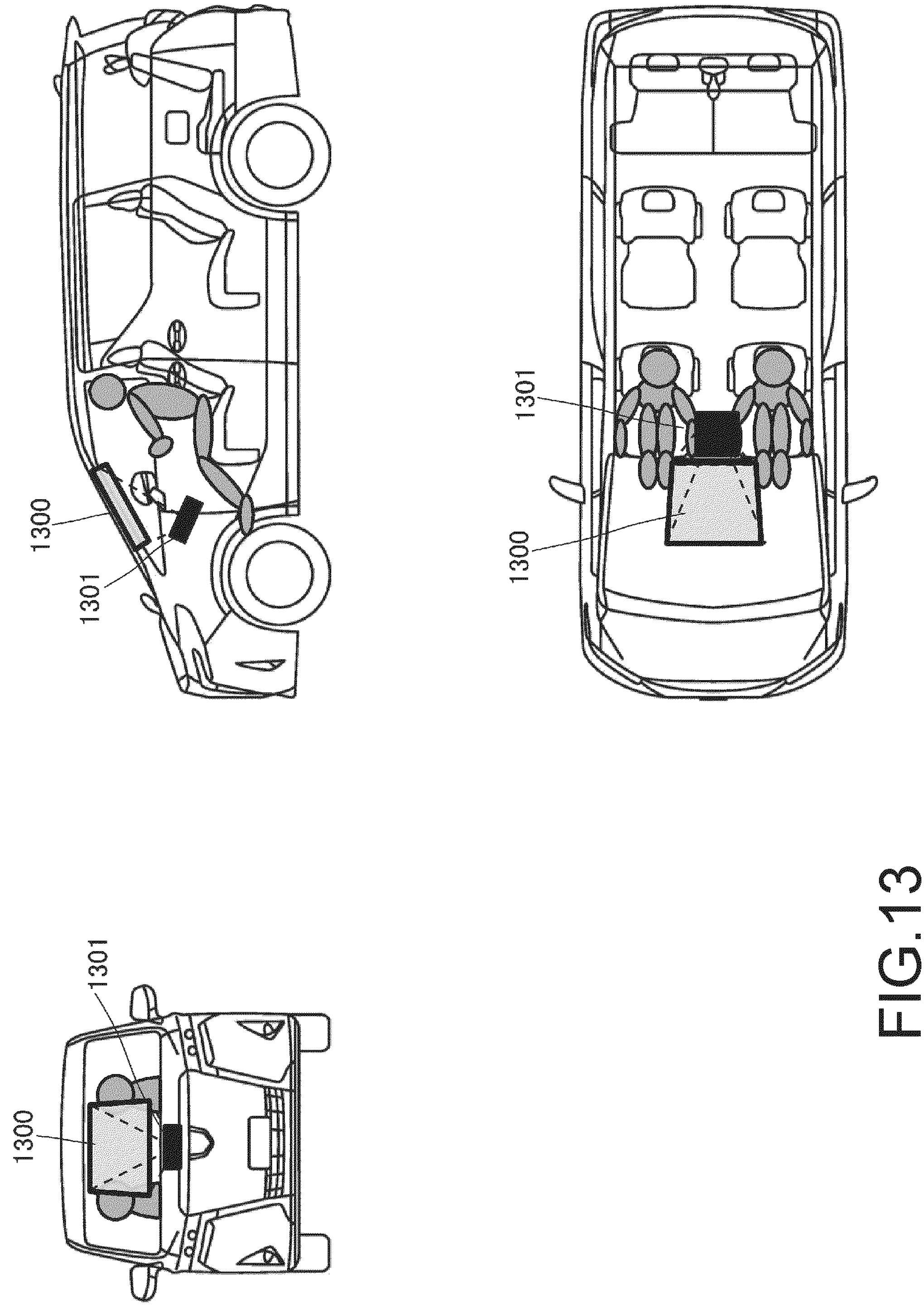


FIG.13

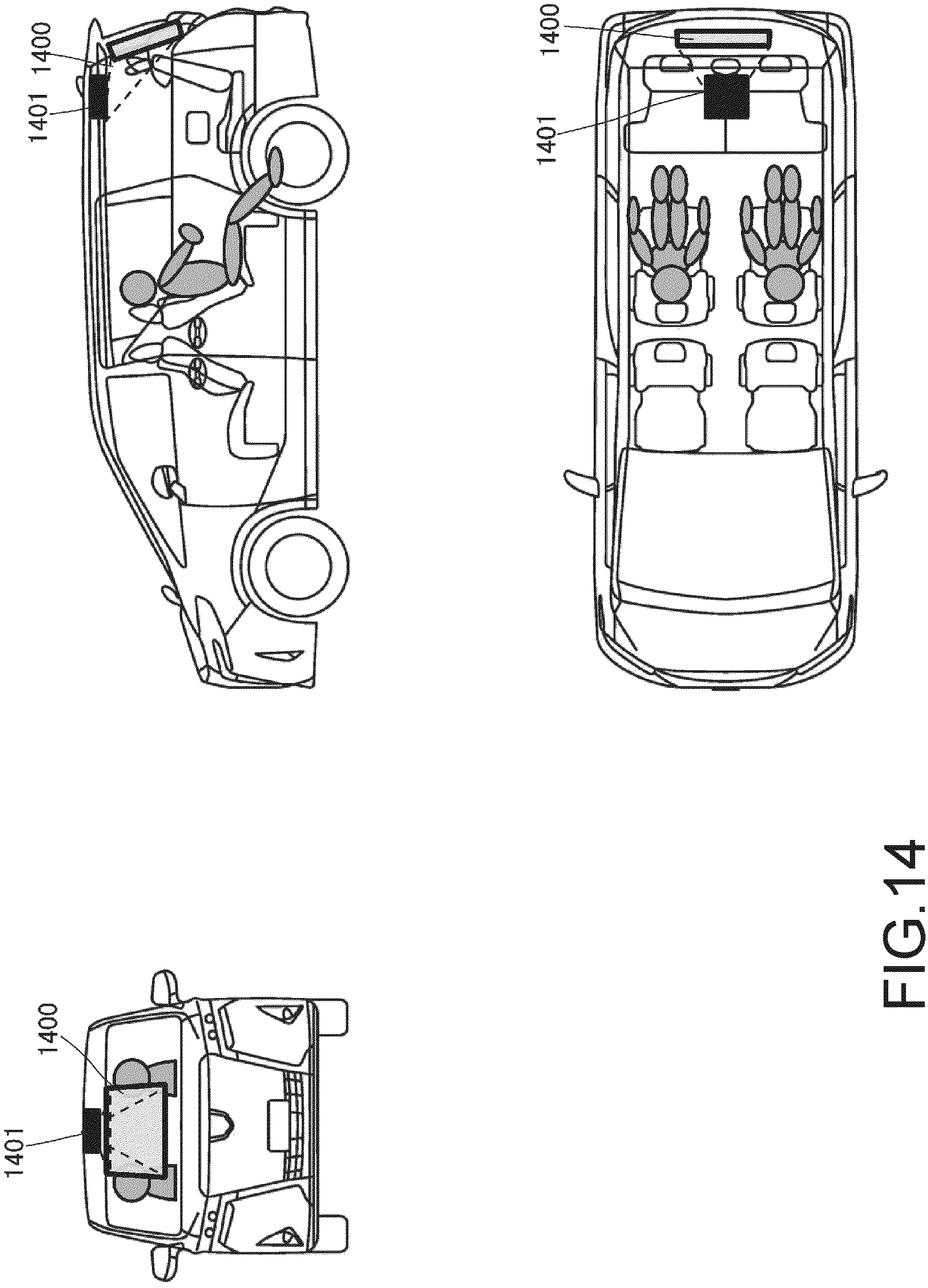


FIG.14

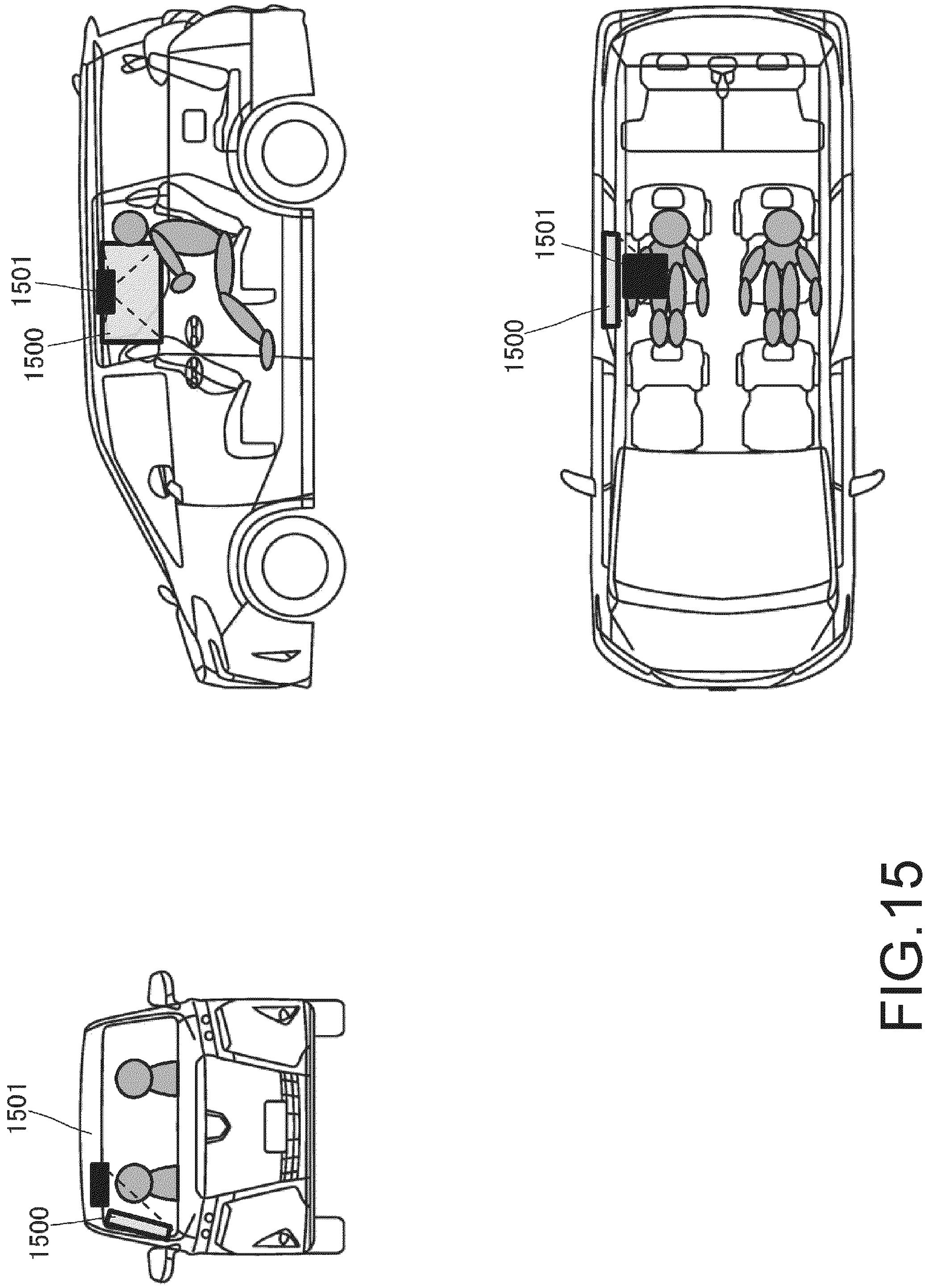


FIG.15

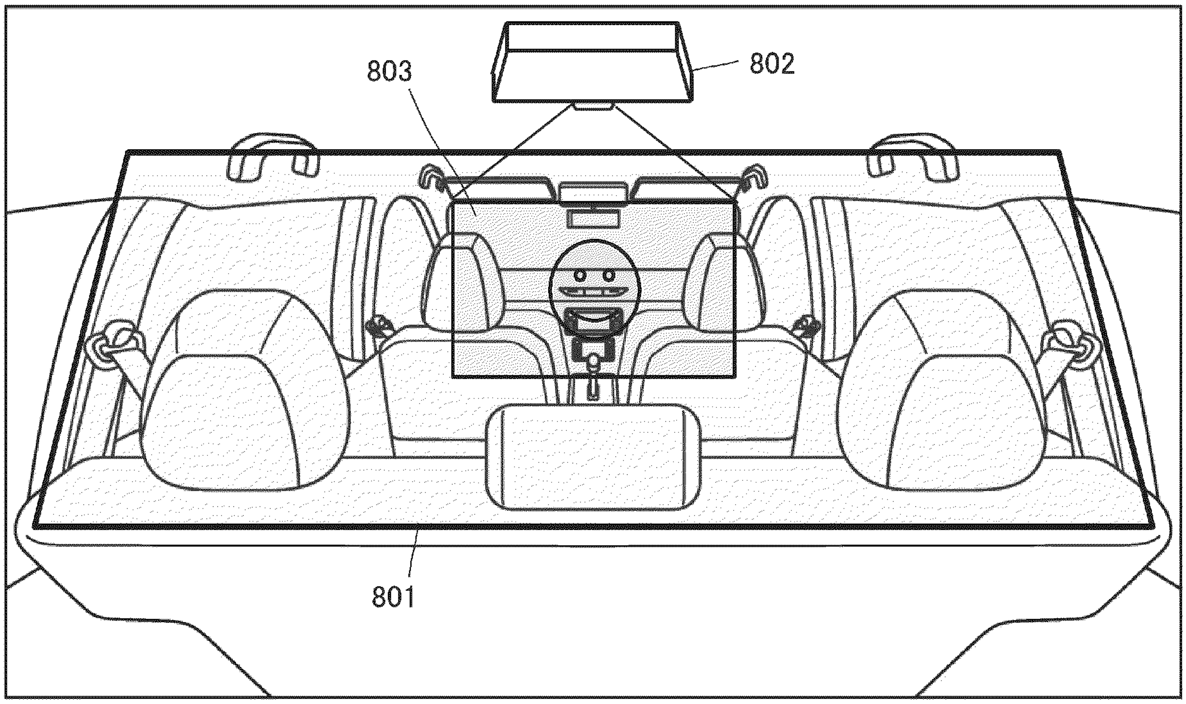


FIG.16

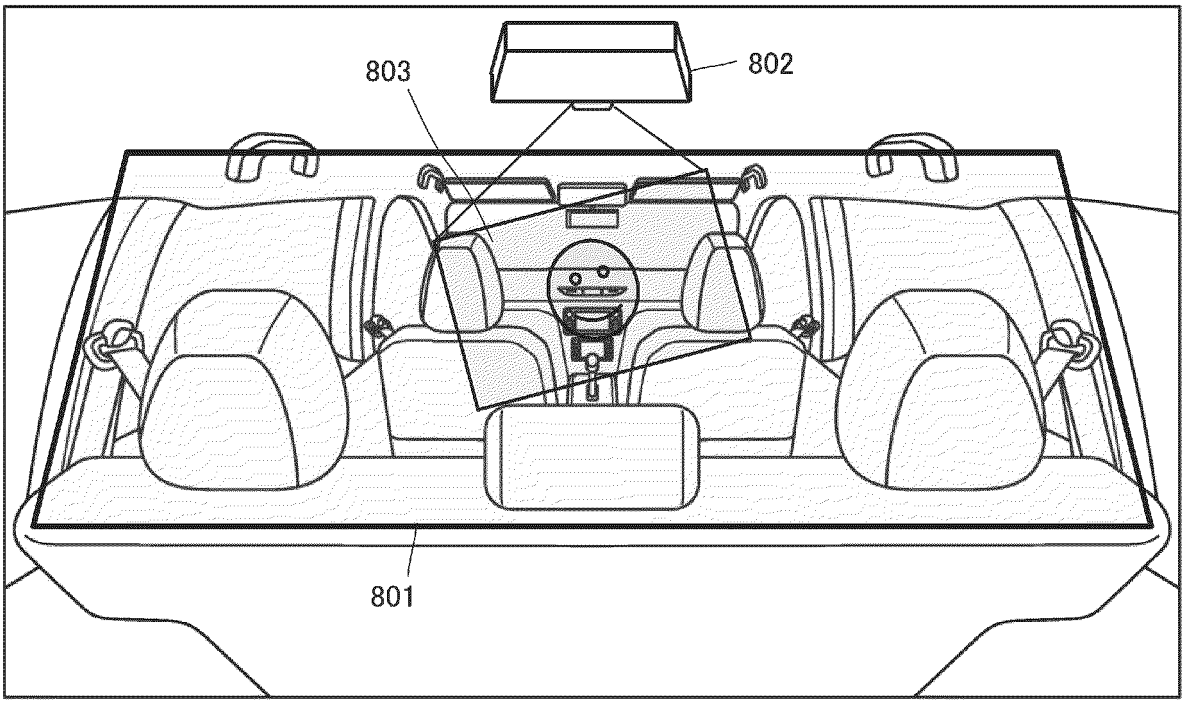


FIG.17

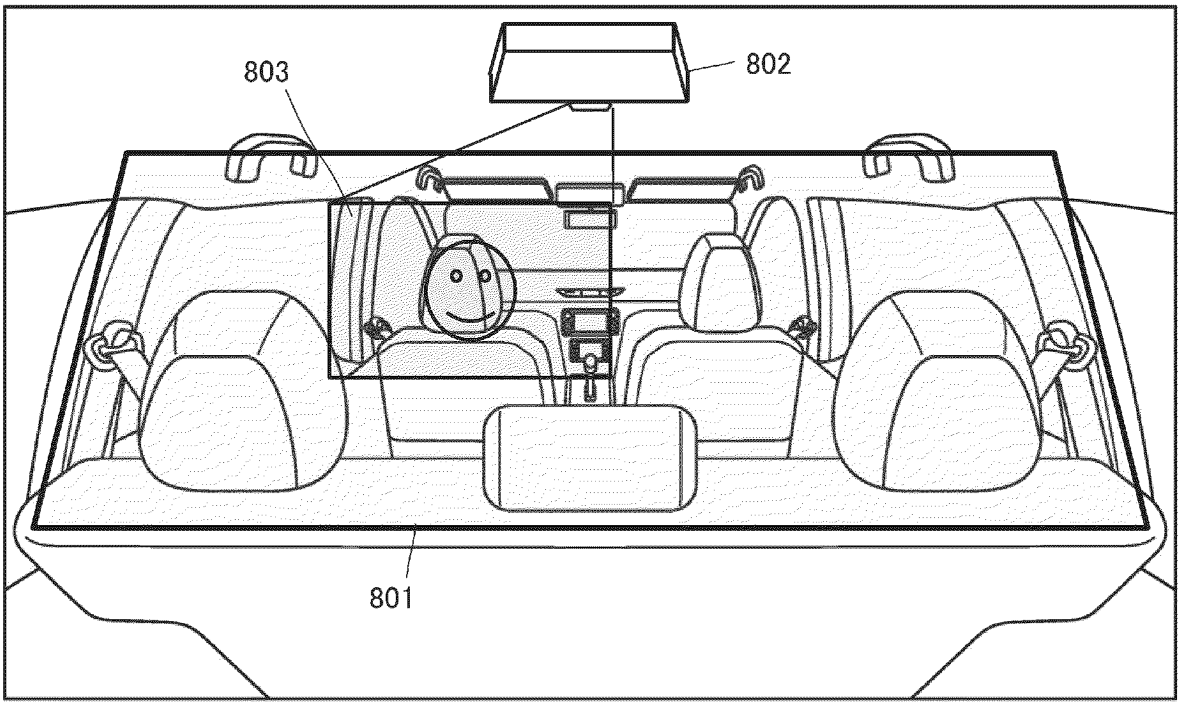


FIG.18

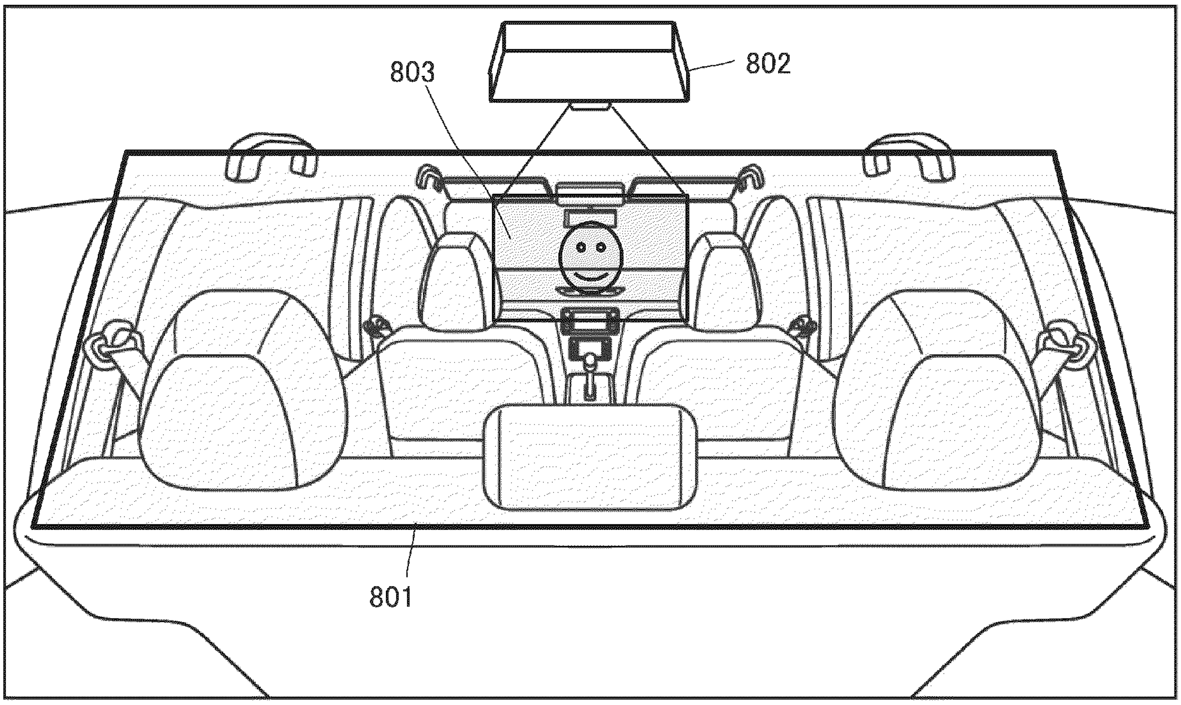


FIG.19

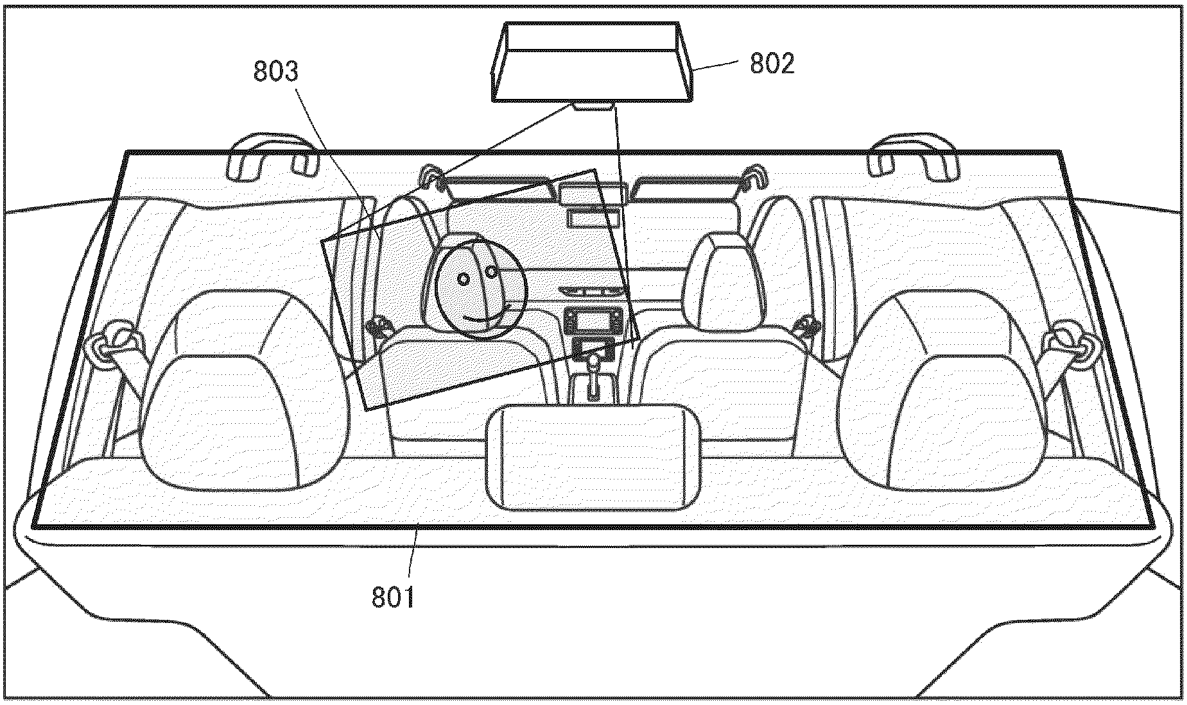


FIG.20

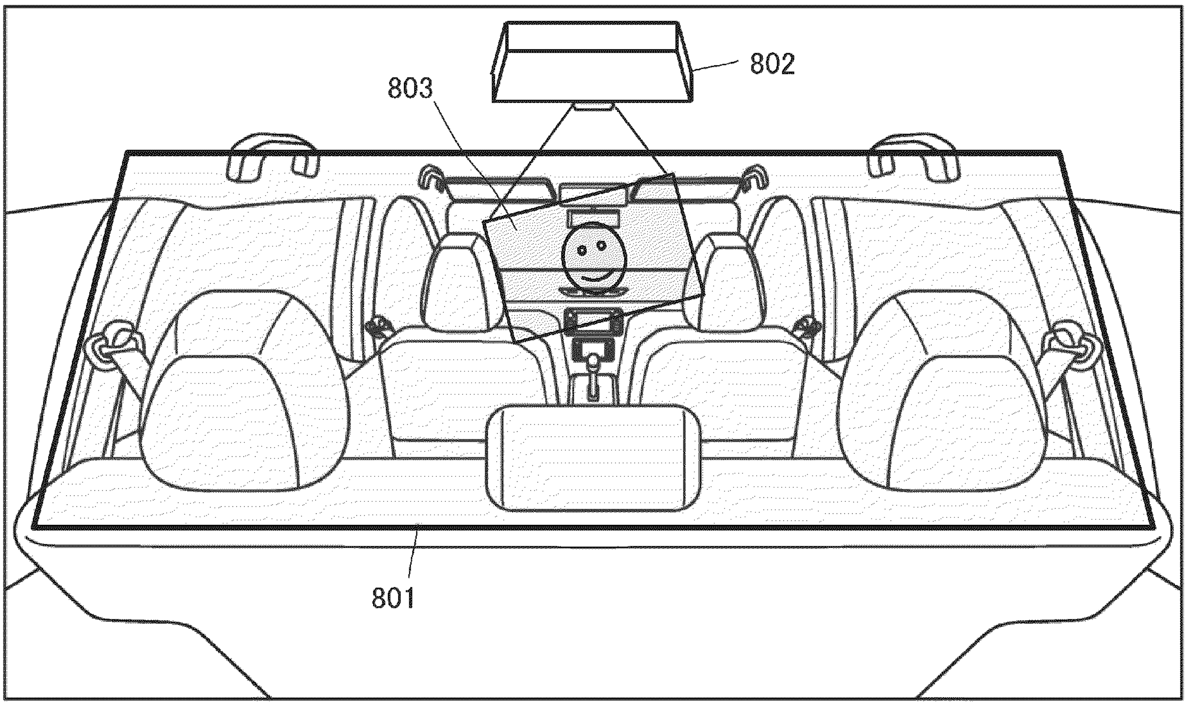


FIG.21

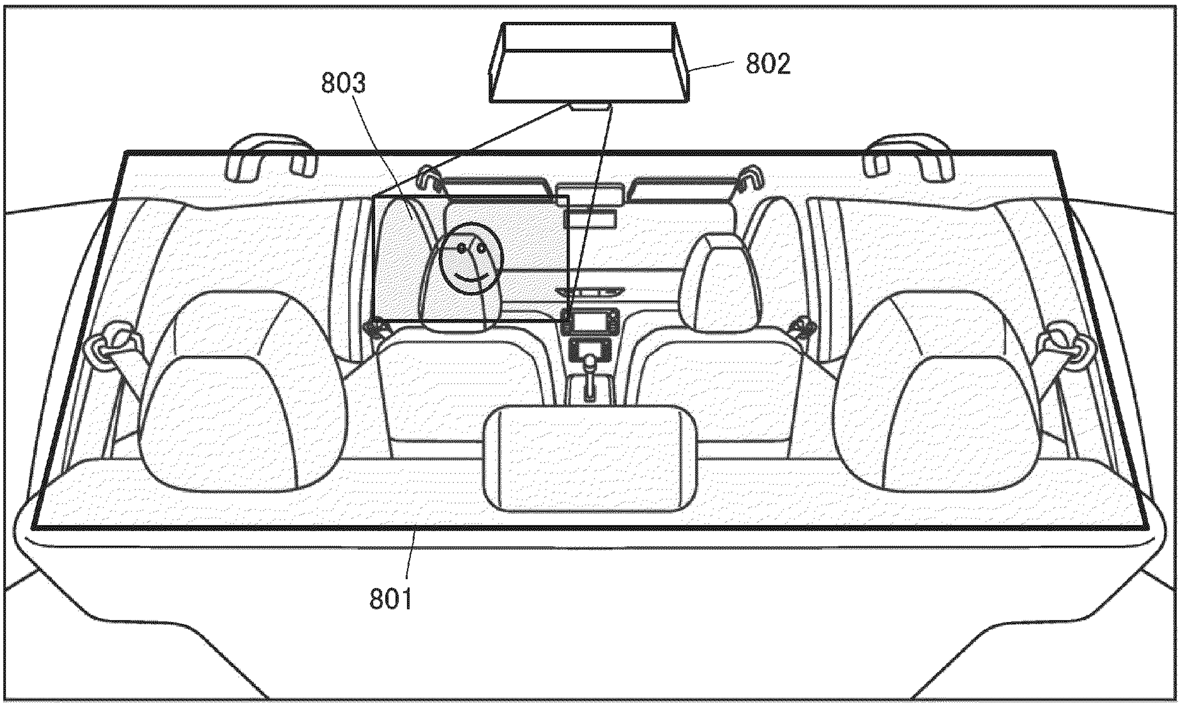


FIG.22

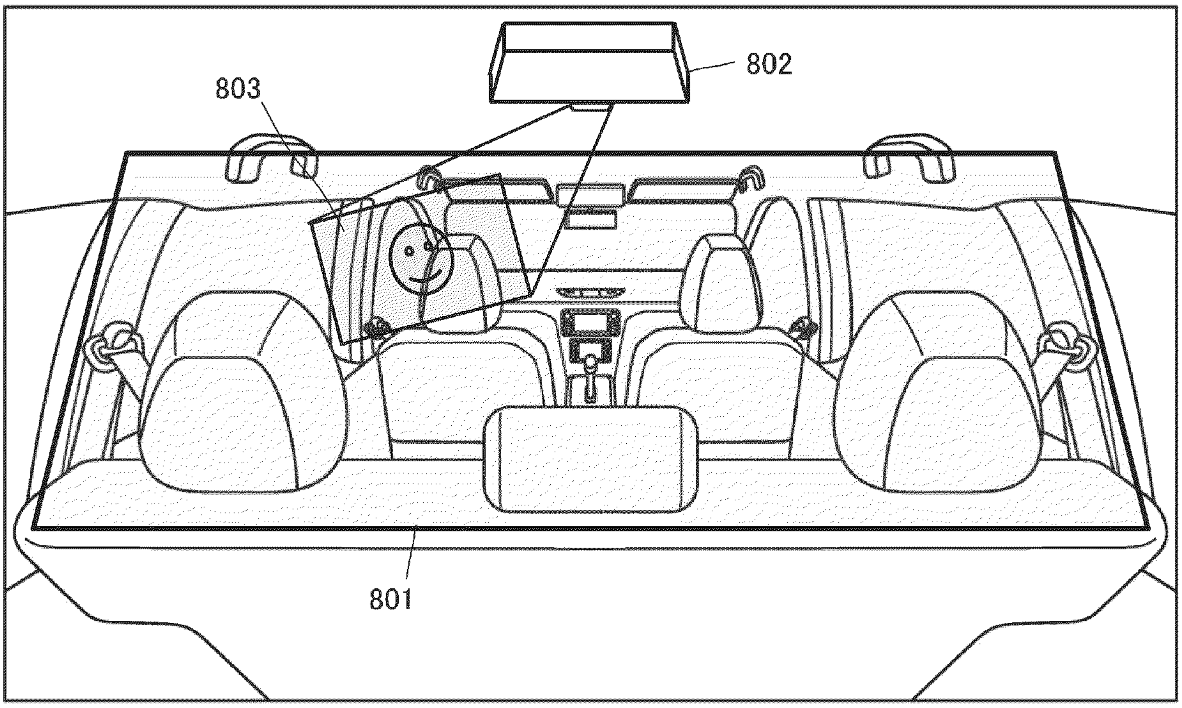


FIG.23

**INTERNATIONAL SEARCH REPORT**

International application No. **PCT/JP2023/017212**

**A. CLASSIFICATION OF SUBJECT MATTER**

***H04N 5/66***(2006.01)i; ***A61B 5/11***(2006.01)i; ***A61B 10/00***(2006.01)i; ***B60R 11/02***(2006.01)i; ***G03B 21/14***(2006.01)i; ***G09G 5/00***(2006.01)i; ***G09G 5/377***(2006.01)i; ***H04N 5/74***(2006.01)i

FI: H04N5/66 Z; A61B10/00 W; A61B5/11 120; G09G5/377 100; G03B21/14 Z; B60R11/02 C; G09G5/00 510B; G09G5/00 510G; G09G5/00 550C; H04N5/74 Z

According to International Patent Classification (IPC) or to both national classification and IPC

**B. FIELDS SEARCHED**

Minimum documentation searched (classification system followed by classification symbols)

H04N5/66; A61B5/11; A61B10/00; B60R11/00; G03B21/00; G09G5/00; H04N5/74

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | US 2018/0370461 A1 (MAGNA ELECTRONICS INC.) 27 December 2018 (2018-12-27) paragraphs [0012]-[0017], fig. 1-2 | 1-3, 8, 10-13, 15-17<br>4-7, 9, 14 |
| Y | JP 2010-532287 A (CHOI, Yoon-Jung) 07 October 2010 (2010-10-07) paragraphs [0087]-[0097], fig. 5-6 | 1-3, 8, 10-13, 15-17 |
| Y | JP 2020-124950 A (TOYOTA MOTOR CORP.) 20 August 2020 (2020-08-20) paragraphs [0023]-[0024], fig. 3-4 | 1-3, 8, 10-13, 15-17 |
| Y | JP 2020-87050 A (SONY CORP.) 04 June 2020 (2020-06-04) paragraph [0046] | 15 |
| A | WO 2020/195625 A1 (SONY CORP.) 01 October 2020 (2020-10-01) paragraphs [0148]-[0149] | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2023** | **18 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

**INTERNATIONAL SEARCH REPORT**

International application No. **PCT/JP2023/017212**

**C. DOCUMENTS CONSIDERED TO BE RELEVANT**

| Category* | Citation of document, with indication, where appropriate, of the relevant passages | Relevant to claim No. |
|---|---|---|
| A | 磯部良太ほか, 自動走行酔い:車酔いとVR酔いの併発環境に関する検討, 第22回日本バーチャルリアリティ学会大会論文集, 29 September 2017, (Proceedings of the 22nd Annual Conference of the Virtual Reality Society of Japan.), non-official translation (ISOBE, Ryota et al. Autonomous Driving Sickness: A Study on Simultaneous Occurrence Environment of Car Sickness and VR Sickness.) entire text, all drawings | 1-17 |

Form PCT/ISA/210 (second sheet) (January 2015)

**INTERNATIONAL SEARCH REPORT**
**Information on patent family members**

International application No.
**PCT/JP2023/017212**

| Patent document cited in search report | Publication date (day/month/year) | Patent family member(s) | Publication date (day/month/year) |
|---|---|---|---|
| US 2018/0370461 A1 | 27 December 2018 | (Family: none) | |
| JP 2010-532287 A | 07 October 2010 | WO 2008/093983 A1<br>paragraphs [97]-[109], fig. 5-6<br>KR 10-2008-0071760 A | |
| JP 2020-124950 A | 20 August 2020 | US 2020/0247327 A1<br>paragraphs [0031]-[0032], fig. 3-4<br>CN 111516592 A | |
| JP 2020-87050 A | 04 June 2020 | US 2022/0004002 A1<br>paragraph [0075]<br>WO 2020/110848 A1<br>CN 113168798 A | |
| WO 2020/195625 A1 | 01 October 2020 | US 2022/0144172 A1<br>paragraphs [0240]-[0242] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008242251 A
- JP 2012019452 A
- JP 2019174523 A